# EUROPÄISCHE PATENTSCHRIFT

(11) **EP 2 834 643 B1**
(45) Veröffentlichungstag und Bekanntmachung des Hinweises auf die Patenterteilung: **23.10.2019**
(21) Anmeldenummer: 13718522.9
(22) Anmeldetag: 05.04.2013
(51) Int. Cl.: G01N 33/68

(54) **VERFAHREN ZUR BEHANDLUNG VON BLUT, BLUTPRODUKTEN UND ORGANEN**
METHOD FOR TREATING BLOOD, BLOOD PRODUCTS AND ORGANS
PROCÉDÉ DE TRAITEMENT DE SANG, DE PRODUITS SANGUINS ET D'ORGANES

(30) Priorität: 05.04.2012 DE 102012102999; 14.09.2012 DE 102012108598; 14.09.2012 DE 102012108599
(43) Veröffentlichungstag der Anmeldung: 11.02.2015
(60) Teilanmeldung: 17172222.6
(73) Patentinhaber: Forschungszentrum Jülich GmbH, 52425 Jülich (DE)
(72) Erfinder: WILLBOLD, Dieter, 52428 Jülich (DE)
(74) Vertreter: Fitzner, Uwe
(86) Internationale Anmeldenummer: PCT/EP2013/057160
(87) Internationale Veröffentlichungsnummer: WO 2013/150126

(56) Entgegenhaltungen:
- WO-A2-02/081505
- WO-A2-2004/056318
- WO-A2-2006/005706
- WO-A2-2007/047967
- WO-A2-2007/047967
- WO-A2-2010/062570
- WO-A2-2011/147797
- US-A1- 2005 158 306
- ZHANG G ET AL: "Multiple-Peptide Conjugates for Binding ss-Amyloid Plaques of Alzheimer's Disease", BIOCONJUGATE CHEMISTRY, ACS, WASHINGTON, DC, US, Bd. 14, 1. Januar 2003 (2003-01-01), Seiten 86-92, XP002458160, ISSN: 1043-1802, DOI: 10.1021/BC025526I
- SIDHARTHA M. CHAFEKAR ET AL: "Branched KLVFF Tetramers Strongly Potentiate Inhibition of [beta]-Amyloid Aggregation", CHEMBIOCHEM, Bd. 8, Nr. 15, 15. Oktober 2007 (2007-10-15), Seiten 1857-1864, XP055052606, ISSN: 1439-4227, DOI: 10.1002/cbic.200700338
- STAINS CLIFF I ET AL: "Molecules that target beta-amyloid", CHEMMEDCHEM, WILEY - VCH VERLAG., WEINHEIM, DE, Bd. 2, Nr. 12, 1. Dezember 2007 (2007-12-01), Seiten 1675-1692, XP009107501, ISSN: 1860-7179, DOI: 10.1002/CMDC.200700140
- SUSANNE AILEEN FUNKE ET AL: "Mirror image phage display-a method to generate d-peptide ligands for use in diagnostic or therapeutical applications", MOLECULAR BIOSYSTEMS, Bd. 5, Nr. 8, 1. Januar 2009 (2009-01-01), Seite 783, XP055069250, ISSN: 1742-206X, DOI: 10.1039/b904138a
- THOMAS VAN GROEN ET AL: "Reduction of Alzheimer's Disease Amyloid Plaque Load in Transgenic Mice by D3, a D-Enantiomeric Peptide Identified by Mirror Image Phage Display", CHEMMEDCHEM, Bd. 3, Nr. 12, 15. Dezember 2008 (2008-12-15), Seiten 1848-1852, XP055239322, DE ISSN: 1860-7179, DOI: 10.1002/cmdc.200800273
- SUSANNE AILEEN FUNKE ET AL: "-Enantiomeric Peptide D3 Improves the Pathology and Behavior of Alzheimer's Disease Transgenic Mice", ACS CHEMICAL NEUROSCIENCE, Bd. 1, Nr. 9, 15. September 2010 (2010-09-15), Seiten 639-648, XP055239332, US ISSN: 1948-7193, DOI: 10.1021/cn100057j
- Hongmei Liu ET AL: "Transport of Alzheimer Disease Amyloid-b-Binding d-Amino Acid Peptides across an In Vitro Blood-Brain Barrier Model", , 2. Dezember 2009 (2009-12-02), XP055239347, Gefunden im Internet: URL:http://online.liebertpub.com/doi/pdf/1 0.1089/rej.2009.0926 [gefunden am 2016-01-07]
- RANJINI K. SUNDARAM ET AL: "Novel Detox Gel Depot Sequesters [beta]-Amyloid Peptides in a Mouse Model of Alzheimer's Disease", INTERNATIONAL JOURNAL OF PEPTIDE RESEARCH AND THERAPEUTICS, Bd. 18, Nr. 2, 23. November 2011 (2011-11-23), Seiten 99-106, XP055070073, ISSN: 1573-3149, DOI: 10.1007/s10989-011-9283-7
- FUNKE SUSANNE AILEEN ET AL: "Peptides for Therapy and Diagnosis of Alzheimer's Disease", CURRENT PHARMACEUTICAL DESIGN, BENTHAM SCIENCE PUBLISHERS, NL, Bd. 18, Nr. 6, 1. Februar 2012 (2012-02-01), Seiten 755-767, XP009170739, ISSN: 1381-6128
- ANDREAS MÜLLER-SCHIFFMANN ET AL: "Combining Independent Drug Classes into Superior, Synergistically Acting Hybrid Molecules", ANGEWANDTE CHEMIE INTERNATIONAL EDITION, Bd. 49, Nr. 46, 8. November 2010 (2010-11-08), Seiten 8743-8746, XP055083717, ISSN: 1433-7851, DOI: 10.1002/anie.201004437
- DIRK BARTNIK ET AL: "Differently selected D-enantiomeric peptides act on different A.beta. species", REJUVENATION RESEARCH, MARY ANN LIEBERT, NEW ROCHELLE, NY, US, Bd. 13, Nr. 2-3, 12. Mai 2010 (2010-05-12) , Seiten 202-205, XP002654152, ISSN: 1549-1684, DOI: 10.1089/REJ.2009.0924 [gefunden am 2009-12-02]
- ZHANG G ET AL: "Multiple-Peptide Conjugates for Binding ss-Amyloid Plaques of Alzheimer's Disease", BIOCONJUGATE CHEMISTRY, ACS, WASHINGTON, DC, US, vol. 14, 1 January 2003 (2003-01-01), pages 86-92, XP002458160, ISSN: 1043-1802, DOI: 10.1021/BC025526I
- SIDHARTHA M. CHAFEKAR ET AL: "Branched KLVFF Tetramers Strongly Potentiate Inhibition of [beta]-Amyloid Aggregation", CHEMBIOCHEM, vol. 8, no. 15, 15 October 2007 (2007-10-15), pages 1857-1864, XP055052606, ISSN: 1439-4227, DOI: 10.1002/cbic.200700338
- STAINS CLIFF I ET AL: "Molecules that target beta-amyloid", CHEMMEDCHEM, WILEY - VCH VERLAG., WEINHEIM, DE, vol. 2, no. 12, 1 December 2007 (2007-12-01), pages 1675-1692, XP009107501, ISSN: 1860-7179, DOI: 10.1002/CMDC.200700140
- SUSANNE AILEEN FUNKE ET AL: "Mirror image phage display-a method to generate d-peptide ligands for use in diagnostic or therapeutical applications", MOLECULAR BIOSYSTEMS, vol. 5, no. 8, 1 January 2009 (2009-01-01) , page 783, XP055069250, ISSN: 1742-206X, DOI: 10.1039/b904138a
- THOMAS VAN GROEN ET AL: "Reduction of Alzheimer's Disease Amyloid Plaque Load in Transgenic Mice by D3, a D-Enantiomeric Peptide Identified by Mirror Image Phage Display", CHEMMEDCHEM, vol. 3, no. 12, 15 December 2008 (2008-12-15), pages 1848-1852, XP055239322, DE ISSN: 1860-7179, DOI: 10.1002/cmdc.200800273
- SUSANNE AILEEN FUNKE ET AL: "-Enantiomeric Peptide D3 Improves the Pathology and Behavior of Alzheimer's Disease Transgenic Mice", ACS CHEMICAL NEUROSCIENCE, vol. 1, no. 9, 15 September 2010 (2010-09-15), pages 639-648, XP055239332, US ISSN: 1948-7193, DOI: 10.1021/cn100057j
- Hongmei Liu ET AL: "Transport of Alzheimer Disease Amyloid-b-Binding d-Amino Acid Peptides across an In Vitro Blood-Brain Barrier Model", , 2 December 2009 (2009-12-02), XP055239347, Retrieved from the Internet: URL:http://online.liebertpub.com/doi/pdf/1 0.1089/rej.2009.0926 [retrieved on 2016-01-07]
- Katja Wiesehan ET AL: "Selection of D-Amino-Acid Peptides That Bind to Alzheimer's Disease Amyloid Peptide A[beta]142 by Mirror Image Phage Display", ChemBioChem, vol. 4, no. 8, 4 August 2003 (2003-08-04), pages 748-753, XP055069247, ISSN: 1439-4227, DOI: 10.1002/cbic.200300631

### Sequence Listing

<110> Forschungszentrum Juelich GmbH
<120> Verfahren zur Behandlung von Blut, Blutprodukten und Organen
<130> FZJ1205PCT
<160> 79
<170> patentin version 3.5
<210> 1
   <211> 12
   <212> PRT
   <213> Artificial Sequence
<220>
   <223> D-Peptid
<400> 1
   Arg Pro Arg Thr Arg Leu Arg Thr His Gln Asn Arg
   1               5                   10
<210> 2
   <211> 16
   <212> PRT
   <213> artificial sequence
<220>
   <223> D-Peptid
<400> 2
   Lys Gln His His Val Glu Tyr Gly Ser Asp His Arg Phe Glu Ala Asp
   1               5                   10                  15
<210> 3
   <211> 8
   <212> PRT
   <213> artificial sequence
<220><223> D-Peptid
<400> 3
   Ser His Tyr Arg His Ile Ser Pro
   1               5
<210> 4
   <211> 12
   <212> PRT
   <213> artificial sequence
<220><223> D-Peptid
<400> 4
   Gly Ile Ser Trp Gln Gln Ser His His Leu Val Ala
   1               5                   10
<210> 5
   <211> 8
   <212> PRT
   <213> artificial sequence
<220><223> D-Peptid
<400> 5
   Pro Arg Thr Arg Leu His Thr His
   1 5
<210> 6
   <211> 12
   <212> PRT
   <213> artificial sequence
<220><223> D-Peptid
<400> 6
   Gln Ser His Tyr Arg His Ile Ser Pro Ala Gln Val
   1 5 10
<210> 7
   <211> 12
   <212> PRT
   <213> artificial sequence
<220><223> D-Peptid
<400> 7
   Gln Ser His Tyr Arg His Ile Ser Pro Asp Gln Val
   1 5 10
<210> 8
   <211> 11
   <212> PRT
   <213> artificial sequence
<220><223> D-Peptid
<400> 8
   Gln Ser His Tyr Arg His Ile Ser Pro Ala Arg
   1 5 10
<210> 9
   <211> 12
   <212> PRT
   <213> artificial sequence
<220><223> D-Peptid
<400> 9
   Lys Ser His Tyr Arg His Ile Ser Pro Ala Lys Val
   1 5 10
<210> 10
   <211> 12
   <212> PRT
   <213> artificial sequence
<220><223> D-Peptid
<400> 10
   Arg Pro Arg Thr Arg Leu His Thr His Arg Asn Arg
   1               5                   10
<210> 11
   <211> 12
   <212> PRT
   <213> artificial sequence
<220><223> D-Peptid
<400> 11
   Arg Pro Arg Thr Arg Leu His Thr His Arg Thr Glu
   1               5                   10
<210> 12
   <211> 12
   <212> PRT
   <213> artificial sequence
<220><223> D-Peptid
<400> 12
   Lys Pro Arg Thr Arg Leu His Thr His Arg Asn Arg
   1               5                   10
<210> 13
   <211> 24
   <212> PRT
   <213> artificial sequence
<220><223> D-Peptid
<400> 13
   Arg Pro Arg Thr Arg Leu His Thr His Arg Asn Arg Arg Pro Arg Thr
   1               5                   10                  15
   Arg Leu His Thr His Arg Asn Arg
               20
<210> 14
   <211> 27
   <212> PRT
   <213> artificial sequence
<220><223> D-Peptid
<400> 14
   Arg Pro Arg Thr Arg Leu His Thr His Arg Asn Arg Asn Trp Asn Arg
   1 5 10 15
   Pro Arg Thr Arg Leu His Thr His Arg Asn Arg
   20 25
<210> 15
   <211> 24
   <212> PRT
   <213> artificial sequence
<220><223> D-Peptid
<220>
   <223> doppel-D3-freie-Ntermini (rprtrlhthrnr)2-PEG3
<400> 15
   Arg Pro Arg Thr Arg Leu His Thr His Arg Asn Arg Arg Pro Arg Thr
   1 5 10 15
   Arg Leu His Thr His Arg Asn Arg
   20
<210> 16
   <211> 24
   <212> PRT
   <213> artificial sequence
<220><223> D-Peptid
<220>
   <223> doppel-D3-freie-Ctermini: PEG5-(rprtrlhthrnr)2
<400> 16
   Arg Pro Arg Thr Arg Leu His Thr His Arg Asn Arg Arg Pro Arg Thr
   1 5 10 15
   Arg Leu His Thr His Arg Asn Arg
   20
<210> 17
   <211> 11
   <212> PRT
   <213> artificial sequence
<220><223> D-Peptid
<400> 17
   Asp Ala Glu Phe Arg His Asp Ser Gly Tyr Glu
   1 5 10
<210> 18
   <211> 12
   <212> PRT
   <213> artificial sequence
<220><223> D-Peptid
<400> 18
   His His Gly His Ser Pro Asn Val Ser Gln Val Arg
   1               5                   10
<210> 19
   <211> 12
   <212> PRT
   <213> artificial sequence
<220><223> D-Peptid
<400> 19
   Gly Ser Phe Ser Thr Gln Val Gly Ser Leu His Arg
   1               5                   10
<210> 20
   <211> 11
   <212> PRT
   <213> artificial sequence
<220><223> D-Peptid
<400> 20
   His Thr Gly Thr Gln Ser Tyr Val Pro Arg Leu
   1               5                   10
<210> 21
   <211> 12
   <212> PRT
   <213> artificial sequence
<220><223> D-Peptid
<400> 21
   Thr Leu Ala Tyr Ala Arg Ala Tyr Met Val Ala Pro
   1               5                   10
<210> 22
   <211> 12
   <212> PRT
   <213> artificial sequence
<220><223> D-Peptid
<400> 22
   Thr Leu Ala Tyr Ala Arg Ala Tyr Met Val Ala Pro
   1               5                   10
<210> 23
   <211> 12
   <212> PRT
   <213> artificial sequence
<220><223> D-Peptid
<400> 23
   Ala Thr Pro Gln Asn Asp Leu Lys Thr Phe Pro His
   1               5                   10
<210> 24
   <211> 12
   <212> PRT
   <213> artificial sequence
<220><223> D-Peptid
<400> 24
   Thr Gln Pro Glu Thr Asp Leu Leu Arg Val Gln Phe
   1               5                   10
<210> 25
   <211> 11
   <212> PRT
   <213> artificial sequence
<220><223> D-Peptid
<400> 25
<210> 26
   <211> 12
   <212> PRT
   <213> artificial sequence
<220><223> D-Peptid
<400> 26
   Thr Phe Leu Glu Thr Gly Pro Ile Tyr Ala Asp Gly
   1               5                   10
<210> 27
   <211> 12
   <212> PRT
   <213> artificial sequence
<220><223> D-Peptid
<400> 27
   Leu Val Pro Pro Thr His Arg His Trp Pro Val Thr
   1 5 10
<210> 28
   <211> 12
   <212> PRT
   <213> artificial sequence
<220><223> D-Peptid
<400> 28
   Ala Pro Pro Gly Asn Trp Arg Asn Tyr Leu Met Pro
   1 5 10
<210> 29
   <211> 12
   <212> PRT
   <213> artificial sequence
<220><223> D-Peptid
<400> 29
   Asp Asn Tyr Ser Asn Tyr Val Pro Gly Thr Lys Pro
   1 5 10
<210> 30
   <211> 12
   <212> PRT
   <213> artificial sequence
<220><223> D-Peptid
<400> 30
   Ser Val Ser Val Gly Met Lys Pro Ser Pro Arg Pro
   1 5 10
<210> 31
   <211> 12
   <212> PRT
   <213> artificial sequence
<220><223> D-Peptid
<400> 31
   Ser Leu Pro Asn Pro Phe Ser Val Ser Ser Phe Gly
   1 5 10
<210> 32
   <211> 12
   <212> PRT
   <213> artificial sequence
<220><223> D-Peptid
<400> 32
   Tyr Val His Asn Pro Tyr His Leu Pro Asn Pro Pro
   1 5 10
<210> 33
   <211> 12
   <212> PRT
   <213> artificial sequence
<220><223> D-Peptid
<400> 33
<210> 34
   <211> 12
   <212> PRT
   <213> artificial sequence
<220><223> D-Peptid
<400> 34
   Gly Ala Thr Met Lys Lys Met Asp Asp His Thr Val
   1 5 10
<210> 35
   <211> 12
   <212> PRT
   <213> artificial sequence
<220><223> D-Peptid
<400> 35
   Leu Gly Lys Thr Gln Lys Leu Ser Asp Ala His Ser
   1 5 10
<210> 36
   <211> 12
   <212> PRT
   <213> artificial sequence
<220><223> D-Peptid
<400> 36
   Asp Asp Gln Ala Arg Pro Tyr Met Ala Tyr Gly Pro
   1 5 10
<210> 37
   <211> 12
   <212> PRT
   <213> artificial sequence
<220><223> D-Peptid
<400> 37
   Gly Asp Thr Trp Val Asn Met Val Ser Met Val His
   1               5                   10
<210> 38
   <211> 12
   <212> PRT
   <213> artificial sequence
<220><223> D-Peptid
<400> 38
   Gly Tyr Thr Trp Val Asn Met Val Ser Met Val His
   1               5                   10
<210> 39
   <211> 12
   <212> PRT
   <213> artificial sequence
<220><223> D-Peptid
<400> 39
   Trp Thr Asn Thr Val Ala Arg Leu Ala Thr Pro Tyr
   1               5                   10
<210> 40
   <211> 12
   <212> PRT
   <213> artificial sequence
<220><223> D-Peptid
<400> 40
   Gln Thr Gln Ala Leu Tyr His Ser Arg Gln Val His
   1               5                   10
<210> 41
   <211> 12
   <212> PRT
   <213> artificial sequence
<220><223> D-Peptid
<400> 41
   Asn Ser Gln Thr Gln Thr Leu His Leu Phe Pro His
   1               5                   10
<210> 42
   <211> 12
   <212> PRT
   <213> artificial sequence
<220><223> D-Peptid
<400> 42
   His Asn Thr Ser Ala Asn Ile Leu His Ser Ser His
   1 5 10
<210> 43
   <211> 12
   <212> PRT
   <213> artificial sequence
<220><223> D-Peptid
<400> 43
   Ser His Ile Asn Pro Thr Ser Phe Trp Pro Ala Pro
   1 5 10
<210> 44
   <211> 12
   <212> PRT
   <213> artificial sequence
<220><223> D-Peptid
<400> 44
   Thr Phe Ser Asn Pro Leu Tyr Met Trp Pro Arg Pro
   1 5 10
<210> 45
   <211> 12
   <212> PRT
   <213> artificial sequence
<220><223> D-Peptid
<400> 45
   Gly Pro Ser Pro Phe Asn Pro Gln Pro Thr Pro Val
   1 5 10
<210> 46
   <211> 12
   <212> PRT
   <213> artificial sequence
<220><223> D-Peptid
<400> 46
   Phe Ser Asp His Lys Ser Pro Thr Pro Pro Pro Arg
   1 5 10
<210> 47
   <211> 12
   <212> PRT
   <213> artificial sequence
<220><223> D-Peptid
<400> 47
   Ser Thr Ser Val Tyr Pro Pro Pro Pro Ser Ala Trp
   1 5 10
<210> 48
   <211> 12
   <212> PRT
   <213> artificial sequence
<220><223> D-Peptid
<400> 48
   Tyr Gly Leu Pro Thr Gln Ala Asn Ser Met Gln Leu
   1 5 10
<210> 49
   <211> 12
   <212> PRT
   <213> artificial sequence
<220><223> D-Peptid
<400> 49
   His Asn Arg Thr Asp Asn Thr Tyr Ile Arg Pro Thr
   1 5 10
<210> 50
   <211> 12
   <212> PRT
   <213> artificial sequence
<220><223> D-Peptid
<400> 50
   Leu Gln Gln Pro Leu Gly Asn Asn Arg Pro Asn Ser
   1 5 10
<210> 51
   <211> 12
   <212> PRT
   <213> artificial sequence
<220><223> D-Peptid
<400> 51
   Lys Pro Glu Asp Ser Ala Ala Tyr Pro Gln Asn Arg
   1               5                   10
<210> 52
   <211> 13
   <212> PRT
   <213> artificial sequence
<220><223> D-Peptid
<400> 52
   Arg Pro Glu Asp Ser Val Ile Thr Lys Thr Gln Asn Thr
   1               5                   10
<210> 53
   <211> 12
   <212> PRT
   <213> artificial sequence
<220><223> D-Peptid
<400> 53
   Arg Ala Ala Asp Ser Gly cys Thr Pro Thr Lys His
   1               5                   10
<210> 54
   <211> 12
   <212> PRT
   <213> artificial sequence
<220><223> D-Peptid
<400> 54
   Arg Pro Arg Thr Arg Leu His Thr His Arg Asn Thr
   1               5                   10
<210> 55
   <211> 12
   <212> PRT
   <213> artificial sequence
<220><223> D-Peptid
<400> 55
   Arg Pro Arg Thr Arg Leu His Thr His Thr Asn Val
   1               5                   10
<210> 56
   <211> 12
   <212> PRT
   <213> artificial sequence
<220><223> D-Peptid
<400> 56
   Arg Pro Arg Thr Arg Leu His Thr His Thr Asn Arg
   1 5 10
<210> 57
   <211> 12
   <212> PRT
   <213> artificial sequence
<220><223> D-Peptid
<400> 57
   Arg Pro Arg Thr Arg Leu His Thr His Arg Lys Gln
   1 5 10
<210> 58
   <211> 12
   <212> PRT
   <213> artificial sequence
<220><223> D-Peptid
<400> 58
   Arg Pro Arg Thr Arg Leu His Thr Leu Arg Asn Arg
   1 5 10
<210> 59
   <211> 12
   <212> PRT
   <213> artificial sequence
<220><223> D-Peptid
<400> 59
   Arg Arg Arg Ser Pro Leu His Thr His Arg Asn Arg
   1 5 10
<210> 60
   <211> 12
   <212> PRT
   <213> artificial sequence
<220><223> D-Peptid
<400> 60
   Leu Arg Ser Pro Arg Gln Arg Arg Ile Pro Arg Ile
   1 5 10
<210> 61
   <211> 12
   <212> PRT
   <213> artificial sequence
<220><223> D-Peptid
<400> 61
   Arg Lys Arg Gln Leu Arg Met Thr Thr Pro Arg Pro
   1               5                   10
<210> 62
   <211> 11
   <212> PRT
   <213> artificial sequence
<220><223> D-Peptid
<400> 62
   Ser His Tyr Arg His Ile Ser Pro Ala Gln Lys
   1               5                   10
<210> 63
   <211> 24
   <212> PRT
   <213> artificial sequence
<220>
   <223> D-Peptid
<220>
   <223> doppel-D3-freie-Ntermini (rprtrlhthrnr)2
<400> 63
   Arg Pro Arg Thr Arg Leu His Thr His Arg Asn Arg Arg Pro Arg Thr
   1               5                   10                  15
   Arg Leu His Thr His Arg Asn Arg
               20
<210> 64
   <211> 24
   <212> PRT
   <213> artificial sequence
<220>
   <223> D-Peptid
<220>
   <223> doppel-D3-freie-Ctermini: (rprtrlhthrnr)2
<400> 64
   Arg Pro Arg Thr Arg Leu His Thr His Arg Asn Arg Arg Pro Arg Thr
   1 5 10 15
   Arg Leu His Thr His Arg Asn Arg
   20
<210> 65
   <211> 12
   <212> PRT
   <213> Artificial Sequence
<220>
   <223> D-Peptid
<400> 65
   Arg Pro Ile Thr Arg Leu Arg Thr His Gln Asn Arg
   1 5 10
<210> 66
   <211> 12
   <212> PRT
   <213> Artificial Sequence
<220>
   <223> D-Peptid
<400> 66
   Pro Thr Leu His Thr His Asn Arg Arg Arg Arg Arg
   1 5 10
<210> 67
   <211> 12
   <212> PRT
   <213> Artificial Sequence
<220>
   <223> D-Peptid
<400> 67
   Pro Asn His His Arg Arg Arg Arg Arg Thr Thr Leu
   1 5 10
<210> 68
   <211> 12
   <212> PRT
   <213> Artificial Sequence
<220>
   <223> D-Peptid
<400> 68
   Arg Arg Pro Thr Leu Arg His Thr His Asn Arg Arg
   1 5 10
<210> 69
   <211> 9
   <212> PRT
   <213> Artificial Sequence
<220>
   <223> D-Peptid
<400> 69
   Arg Pro Arg Thr Arg Leu Arg Asn Arg
   1 5
<210> 70
   <211> 6
   <212> PRT
   <213> Artificial Sequence
<220>
   <223> D-Peptid
<400> 70
   Arg Pro Arg Thr Arg Leu
   1 5
<210> 71
   <211> 12
   <212> PRT
   <213> Artificial Sequence
<220>
   <223> D-Peptid
<400> 71
   Arg Pro Ile Thr Arg Leu His Thr Asp Arg Asn Arg
   1 5 10
<210> 72
   <211> 12
   <212> PRT
   <213> Artificial Sequence
<220>
   <223> D-Peptid
<400> 72
   Arg Pro Ile Thr Thr Leu Gln Thr His Gln Asn Arg
   1 5 10
<210> 73
   <211> 12
   <212> PRT
   <213> artificial sequence
<220>
   <223> D-Peptid
<400> 73
   Arg Pro Arg Thr Arg Leu His Thr His Arg Asn Arg
   1 5 10
<210> 740
   <211> 12
   <212> PRT
   <213> Artificial Sequence
<220>
   <223> D-Peptid
<400> 74
<210> 75
   <211> 18
   <212> PRT
   <213> Artificial Sequence
<220>
   <223> D-Peptid
<400> 75
   Arg Pro Arg Thr Arg Leu Arg Asn Arg Arg Pro Arg Thr Arg Leu Arg Asn Arg
   1 5 10 15
<210> 76
   <211> 24
   <212> PRT
   <213> Artificial Sequence
<220>
   <223> D-Peptid
<400> 76
<210> 77
   <211> 12
   <212> PRT
   <213> Artificial Sequence
<220>
   <223> D-Peptid
<400> 77
   Ser Gly Trp His Tyr Asn Trp Gln Tyr Trp Trp Lys
   1               5                   10
<210> 78
   <211> 20
   <212> PRT
   <213> Artificial Sequence
<220>
   <223> D-Peptid
<400> 78
   Arg Pro Arg Thr Arg Ser Gly Trp His Tyr Asn Trp Gln Tyr Trp Trp
   1               5                   10                  15
   Lys Arg Asn Arg
               20
<210> 79
   <211> 20
   <212> PRT
   <213> Artificial Sequence
<220>
   <223> D-Peptid
<400> 79
   Pro Thr Leu Ser Gly Trp His Tyr Asn Trp Gln Tyr Trp Trp Lys Arg
   1               5                   10                  15
   Arg Arg Arg Arg
               20

## Beschreibung

Die vorliegende Erfindung betrifft ein Verfahren zur Behandlung (Ex vivo) von Blut, Blutprodukten und Organen zur Prävention der Übertragung von Morbus-Alzheimer und anderen Amyloid-basierten Erkrankungen.

Aufgrund der demographischen Entwicklung in den nächsten Jahrzehnten wird sich die Zahl der Personen, die an altersbedingten Erkrankungen leiden, erhöhen. Hier ist insbesondere die sog. Alzheimer-Krankheit (AD, Alzheimersche Demenz, lateinisch = Morbus Alzheimer) zu nennen.

Ein Merkmal der Alzheimer-Erkrankung sind extrazelluläre Ablagerungen des Amyloid-Beta-Peptids (A-Beta-Peptid, Aß, oder Aß-Peptid). Diese Ablagerung des A-Beta-Peptids in Plaques ist typischerweise in den Gehirnen von AD-Patienten *post mortem* festzustellen. Deshalb werden verschiedene Formen des A-Beta-Peptids - wie z.B. Fibrillen - für die Entstehung und das Fortschreiten der Krankheiten verantwortlich gemacht. Zusätzlich werden seit einigen Jahren die kleinen, frei diffundierbaren A-Beta-Oligomere als hauptsächliche Verursacher der Entstehung und des Fortschritts der AD gesehen.
A-Beta-Monomere, als Bausteine der A-Beta-Oligomeren entstehen im menschlichen Körper ständig und sind vermutlich *per se* nicht toxisch. A-Beta-Monomere können sich in Abhängigkeit von ihrer Konzentration zufällig zusammen lagern. Die Konzentration ist abhängig von ihrer Bildungs- und Abbaurate im Körper. Findet mit zunehmendem Alter eine Erhöhung der Konzentration an A-Beta-Monomeren im Körper statt, ist eine spontane Zusammenlagerung der Monomere zu A-Beta-Oligomeren immer wahrscheinlicher. Die so entstandenen A-Beta-Oligomeren könnten sich analog zu den Prionen vermehren und letztendlich zur Morbus Alzheimer-Krankheit führen.

Ein wichtiger Unterschied zwischen Prävention und Behandlung oder gar Heilung der AD liegt in der Tatsache, dass eine Prävention schon durch die Verhinderung der Bildung der ersten A-Beta-Oligomere erreicht werden kann. Hierzu sind einige, wenige A-Beta-Liganden ausreichend, die wenig affin und selektiv bezüglich der A-Beta-Oligomere sind.

Die Bildung der A-Beta-Oligomere aus vielen Monomeren ist eine Reaktion hoher Ordnung und damit in hoher Potenz von der A-Beta-Monomer-Konzentration abhängig. Somit führt schon eine kleine Verringerung der aktiven A-Beta-Monomer-Konzentration zu einer Verhinderung der Bildung der ersten A-Beta-Oligomeren. Auf diesem Mechanismus basiert die bisherige Prävention.
Bei der Behandlung von AD ist jedoch von einer völlig veränderten Situation auszugehen. Hier liegen nämlich A-Beta-Oligomere oder evtl. auch schon größere Polymere oder Fibrillen vor, die durch die Prionen-ähnliche Vermehrung der Oligomeren entstanden sind. Dies ist jedoch eine Reaktion niederer Ordnung und kaum noch von der A-Beta-Monomer-Konzentration abhängig.

Bisher existiert kein zugelassenes Medikament für eine ursächliche Behandlung der Alzheimerschen Demenz (AD). Typischerweise findet man in den Gehirnen von AD-Patienten *post-mortem* Ablagerungen des sogenannten beta-Amyloid-Peptides (Aß oder A-Beta) in Plaques. Deshalb werden schon lange verschiedene Formen des Aß-Oligomeren, z.B: Fibrillen, für die Entstehung und das Fortschreiten von AD verantwortlich gemacht. Seit wenigen Jahren werden besonders die kleinen, frei diffundierbaren Aß-Oligomere als hauptsächliche Verursacher für die Entstehung und den Fortschritt der AD verantwortlich gemacht. Aß-Monomere entstehen ständig in dem menschlichen Körper und sind vermutlich für sich gesehen nicht toxisch. Es wird spekuliert, ob sich Aß-Monomere abhängig von ihrer Konzentration zufällig und damit mit zunehmendem Alter immer wahrscheinlicher spontan zu Aß-Oligomeren zusammen lagern. Einmal entstandene Aß-Oligomere könnten sich durch eine Prion-ähnlichen Mechanismus vermehren und letzten Endes zur Krankheit führen. Seit einiger Zeit wird diskutiert, ob AD-ähnlich wie Prion-Krankheiten prinzipiell von Mensch zu Mensch übertragbar ist. Ähnliches gilt für alle Amyloid-assoziierten Krankheiten (z.B. Parkinson) zu. Insbesondere eine mögliche Übertragung durch Bluttransfusion, Verabreichung von Blutprodukten und Organtransplantationen könnte ohne geeignete Tests und Präventionsmethoden zu einer massiven Gefährdung der Gesundheit von Empfängern führen. In diesem Zusammenhang wurde in der nicht-wissenschaftlichen Literatur von der vorzeitigen Erkrankung an AD einer transgenen Maus berichtet, nachdem deren komplettes Blut gegen das einer erkrankten Maus ausgetauscht wurde.

Aufgrund dieser Überlegungen sollte es eine Möglichkeit geben, Blut, Blutprodukte und Organe durch (prophylaktische oder präventive) Behandlung von infektiösen Partikeln zu befreien, bzw. diese zu inaktivieren. Es sollte dabei das Ziel sein, toxische Aß-Oligomere vollständig zu entfernen oder zu vernichten, also zu enttoxifizieren und damit deren Prionähnliche Vermehrung zu verhindern.

Aus dem Stand der Technik sind verschiedene Methoden zur Eliminierung von bioschädlichen Stoffen, Biopartikeln, Molekülen und pathologischen Proteinablagerungen bekannt. So gibt es Forschungen, nach welchen Nanomagnete eingesetzt werden, um Blut in wenigen Minuten gezielt von einem Giftstoff zu reinigen. Ebenso ist beschrieben worden, durch direkte Absorption von Lipoproteinen (DALI) LDL-Cholesterin aus dem Blut zu entfernen.
Die Immobilisierung von Antikörpern oder Peptiden wird in der DE 600 26 983 T2 oder US 5,968,820 beschrieben.

Ferner ist aus der DE 102009037015 A1 eine Vorrichtung und ein Verfahren zur Eliminierung von bioschädlichen Stoffen aus Körperflüssigkeiten bekannt. Die Isolation von Zellen, Biopartikeln oder Molekülen aus Flüssigkeiten wird in der DE 102005063175 A1 beschrieben. Ferner ist aus der DE 102005031429 A1 ein Verfahren zur selektiven Bestimmung pathologischer Proteinablagerungen bekannt. Schließlich werden in der DE 102005009909 A1 Verbindungen zur Behandlung von Erkrankungen im Zusammenhang mit fehl gefalteten Proteinen beschrieben.

Die aus dem Stand der Technik bekannten Substanzen verringern die Konzentration von A-Beta-Monomeren und/oder Oligomeren auf verschiedenste Art und Weise. So sind z.B. Gamma-Sekretase-Modulatoren bekannt, die im Tierversuch zur Prävention eingesetzt wurden.
Aus der WO 02/081505 und DE 101 17 281 A1 sind verschiedene Sequenzen von D-Aminosäuren bekannt, die an A-Beta-Peptide binden. Diese Sequenzen der WO 02/081505 aus D-Aminosäuren binden mit einer Dissoziationskonstante K_{D}-Wert von 4 µM an Amyloid-Beta-Peptide.
Aus der WO 2011/147797 sind Hybridverbindungen bekannt, bestehend aus Aminopyrazolen und Peptiden, die A-Beta-Oligomerisation verhindern.

Eine Verwendung dieser Verbindungen zur Aufreinigung von Blut, Blutprodukten und/oder Organen wird jedoch nicht offenbart.

Bei vielen Substanzen, die im Tierversuch positive Ergebnisse gezeigt haben, konnte diese Wirkung in klinischen Studien am Menschen nicht bestätigt werden. In klinischen Studien der Phase II und III dürfen nur mit klar AD diagnostizierte Menschen behandelt werden. Hier reicht eine geringe Verringerung der A-Beta-Monomerkonzentration nicht mehr aus, um größere Mengen an A-Beta-Oligomeren zu verhindern, und/oder Einfluss auf den Krankheitsverlauf zu nehmen.

Bisher wird die Alzheimersche Demenz hauptsächlich durch neuro-psychologische Tests diagnostiziert, durch Experimente an Personen, bei denen die Symptome schon erkannt wurden. Es ist jedoch bekannt, dass A-Beta-Oligomere und die zeitlich darauf folgenden Fibrillen und Plaques bis zu 20 Jahre vor dem Auftreten der Symptome im Gehirn der Patienten entstehen, und schon irreversible Schäden verursacht haben können. Allerdings gibt es bisher noch keine Möglichkeit, die AD vor dem Ausbruch der Symptome zu diagnostizieren.

Ferner offenbart die WO 2010/062570 A2 eine Zusammensetzung zur Behandlung und / oder Vorbeugung von Alzheimer-Krankheit.

Sundaram et al. INTERNATIONAL JOURNAL OF PEPTIDE RESEARCH, 18(2) 2012 betrifft das Retro-inverse Peptid, ffvlk, das künstliche Fibrillen aus Aß mit mäßiger Affinität bindet.

Die WO 2004/056318 A2 offenbart Verfahren zum Behandeln von Amyloid-Krankheit in Menschen durch Entfernen von Amyloidpeptiden aus einer oder mehreren Körperflüssigkeiten.

Die WO 2006/005706 A2 offenbart ein Verfahren zur Vorbeugung oder Behandlung von Alzheimer-Krankheit.

Die US 2005/158306 A1 offenbart ein Verfahren zur Behandlung von Alzheimer-Krankheit.

Die WO 2011/147797 A2 betrifft eine Hybridverbindung auf der Basis von Aminopyrazolderivaten und Peptiden zur Verwendung als Therapeutikum bei der Behandlung von Krankheiten.

Funke et al. CURRENT PHARMACEUTICAL DESIGN, 18(6) 2012 beschreibt Peptide, die für die Diagnose und Therapie von AD entwickelt wurden, und die Vor- und Nachteile von Peptid-Medikamenten.

Die WO 02/081505 A2 offenbart Peptide, die an das Peptid Beta-Amyloid mit hoher Bindungsaffinität binden.

Müller-Schiffmann et al. ANGEW. CHEMIE INTERNATIONAL EDITION, 49(46), 2010 beschreibt Hybridverbindungen, bestehend aus einem organischen D enantiomeren β-sheet-auflösenden Teil und einem, D-enantiomeren Aß erkennenden Peptidteil.

Bartinek et al. REJUVENANT RESEARCH, 13(2-3), 2010 offenbart D-enantiomere Aß-bindende Peptide.

Zhang G. et al. Bioconjugate Chemistry, 14(1), 2003 offenbart, dass die Bildung von β-Amyloid-Plaques bei Alzheimer durch den intermolekularen Kontakt der 5-Aminosäuresequenz KLVFF in β-Amyloid-Peptiden mit einer Größe von 40 bis 43 Resten ausgelöst wird.

Sidhartha et al. ChemBioChem, 8(15), 2007 offenbart ein Dendrimer aus vier Peptiden mit dem Motiv KLVFF.

Stains et al, CHEMMEDCHEM, 2(12) 2007 betrifft das aktuelle Wissen auf in Bezug auf Moleküle, die nachweislich mit oligomeren oder fibrillären Formen des beta-Amyloid-Peptids interagieren.

Die WO 2007/047967 A2 betrifft eine Vorrichtung, die innerhalb eines Patienten mit Alzheimer-Krankheit (AD) platziert wird, um neurotoxische beta-Amyloidpeptide (nt-bAP) aus Körperflüssigkeiten zu extrahieren.

Funke et al. MOLECULAR BIOSYSTEMS, 5(8), 2009 beschreibt einen Phagen-Display-Assay zur Identifizierung neuer potenziell therapeutisch aktiver D-enantiomerer Peptide
Van Groen et al, CHEMMEDCHEM, 3(12), 2008, Funke et al. ACS Chemical Neuroscience, 1(9), 2010, sowie Hongmei et al. 2009 offenbarten, dass das D3 Peptid an A-beta Oligomere bindet und diese entfernt.

Aufgabe der vorliegenden Erfindung ist es nunmehr Blut, Blutprodukte und/oder Organe durch Behandlung von toxischen und/oder infektiösen Partikeln zu befreien bzw. diese zu inaktivieren. Es ist das Ziel die in Blut, Blutprodukten oder Organen vorhandenen Aß-Oligomere, vollständig zu entfernen oder in ungefährliche Formen umzuwandeln.

Gegenstand der Erfindung ist demgemäß Verfahren zur Behandlung außerhalb des menschlichen oder tierischen Körpers (in vitro, ex vivo) von Blut, Blutprodukten und/oder Organen indem Amyloid-Beta-Oligomere entfernt und/oder enttoxifiziert werden dadurch gekennzeichnet, dass die D-enantiomere Verbindung rprtrlhthrnrrprtrlhthrnr (D3D3, SEQ ID NO:13) eingesetzt wird, die an Amyloid-Beta-Oligomere bindet.
Das behandelte Blut kann aus einer Blutbank stammen und/oder nach der Behandlung in einer Blutbank gelagert werden.

Ferner ist es möglich, das erfindungsgemäße Verfahren bei gesunden Personen präventiv anzuwenden. Dabei wird das Blut dieser Personen, die keine AD-Symptome aufweisen, von eventuell vorhandenen Amyloid-beta-Oligomeren befreit, ohne Bezug auf Verfahrensschritte zur therapeutischen Behandlung des menschlichen oder tierischen Körpers.

Die Aß-Oligomere können entfernt werden, indem z.B. dem Blut Beads hinzugefügt werden, die Aß-Oligomere binden oder zurück halten. Zur Behandlung von Blutproben können dafür die Aß-Oligomere bindenden Substanzen an magnetische Beads gebunden werden. Ein Beispiel für Beads sind Dynabeads der Firma Dynal. Dynabeads® M-280 Streptavidin (Dynal A.S., Oslo) sind supramagnetische Mikrokugeln, die mit aufgereinigtem Streptavidin, welches Biotin mit hoher Affinität (K_{D} = 10⁻¹⁵) bindet, kovalent verknüpft sind. Nachfolgend können diese Beads mit den daran gebunden Aß-Oligomeren wieder entfernt werden, z.B. durch Affinitätschromatographie, Filtration, Größenausschluss Sedimentation oder Zentrifugation.

In einer Variante der Erfindung können die Aß-Oligomere entfernt werden, indem das Blut und/oder die Blutprobe über eine Oberfläche geleitet wird, die Aß-Oligomere bindet oder zurück hält. Erfindungsgemäß können die Moleküle, die Aß-Oligomer binden bzw. entoxifizieren (sogenannte Fängermoleküle) auf einem Träger angeordnet sein, über den die flüssige Probe geleitet wird. In einer Variante ist auch für die Fängermoleküle eine Immobilisierung mit Hilfe von Nanomagneten möglich. Ebenso ist die Anordnung der Fängermoleküle in einem Dialyse-System möglich. In einer weiteren Variante können die Fängermoleküle aus einem biokompatiblen Material bestehen. Träger können auch Membranen, Filter, Filterschwämme, Beads, Stäbe, Seile, Säulen und Hohlfasern sein.

In einer weiteren Variante betreffend Organe können die Aß-Oligomere entfernt werden, indem die Fängermölekule über und/oder durch ein Organ geleitet werden (in vitro / ex vivo).

In einer weiteren Variante der Erfindung können Aß-Oligomere inaktiviert werden, indem eine Substanz zugefügt wird, die Aß-Oligomere in nicht-toxische, nicht-amyloidogene, nicht-infektiöse Formen umwandelt.

Als Aß-Oligomere-inaktivierende Substanzen oder als Aß-Oligomere-bindende Substanzen sind alle Aß-Oligimere-modifizierenden Liganden verwendbar, z.B. Aß-Antikörper oder Aß-bindende Peptide. Die einzusetzenden Substanzen sollen eine möglichst hohe Affinität zu Aß-Oligomeren besitzen. Die entsprechende Dissoziationskonstante soll im µM-Bereich, bevorzugt nM-Bereich, insbesondere pM-Bereich oder noch tiefer liegen. Da das Zielmolekül der therapeutischen Behandlung ein Aß-Oligomer und damit natürlicher Weise ein multivalentes Target ist, kann in einer Variante der Erfindung für die Behandlung die einzusetzende Substanz aus mehreren Kopien einer bereits effizient Aß-Oligomer-bindenden Einheit hergestellt werden. Bei Abwesenheit störende Einflüsse (z.B. durch sterische Behinderung) kann ein n-mer einer Aß-Oligomer-bindenden Einheit, die an Aß-Oligomer mit einer Dissoziationskonstante (K_{D}) von mindestens x bindet, eine apparente K von xⁿ erreicht werden. Um dies zu erreichen existieren verschiedene Möglichkeiten: die Aß-Oligomer-bindenden Einheiten (Monomereinheiten) können kovalent oder nicht-kovalent (z.B. eine Biotin-Gruppe oder einen Streptavdin-Tetramer) miteinander verknüpft werden. In einer Variante der Erfindung können beliebig viele Kopien auf der Oberfläche von Beads immobilisiert werden.

Im Sinne der vorliegenden Erfindung bezeichnet der Begriff A-Beta-Oligomere sowohl A-Beta-Aggregate als auch A-Beta-Oligomere und auch kleine frei diffundierende A-Beta-Oligomere. Oligomer im Sinne der Erfindung ist ein Polymer gebildet aus 2, 3, 4, 5, 6, 7, 8, 9, 10, 11, 12, 13, 14, 15, 16, 17, 18, 19 oder 20 Monomeren oder Vielfachen davon.

Das vorliegende Verfahren wird außerhalb des menschlichen oder tierischen Körpers durchgeführt. Die hierfür verwendeten Begriffe sind in vitro oder ex vivo.

In einer Ausführung handelt es sich bei den Polymeren um Peptide. Diese bestehen aus D-Aminosäuren.

Im Sinne der vorliegenden Erfindung bedeutet der Begriff "im Wesentlichen aus D-Aminosäuren", dass die einzusetzenden Monomere mindestens 60%, bevorzugt 75%, 80%, besonders bevorzugt 85%, 90%, 95%, insbesondere 96%, 97%, 98%, 99%, 100% aus D-Aminosäuren aufgebaut sind.

In einer Variante werden Monomere eingesetzt, die an ein A-Beta-Monomer und/oder A-Beta-Oligomere und/oder Fibrillen des A-Beta-Peptids mit einer Dissoziationskonstante (K_{D}-Wert) von höchstens 500 µM, bevorzugt 250, 100, 50 µM, besonders bevorzugt 25, 10, 6 µM, insbesondere 4 µM bindet.

Das Polymer enthält 2, 3, 4, 5, 6, 7, 8, 9, 10 oder mehr der oben beschriebenen Monomere.

In einer weiteren nicht erfindungsgemäßen Ausführung werden die Monomere ausgewählt aus der Gruppe bestehend aus:
SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 und SEQ ID NO:79 sowie Homologe davon.

In einer weiteren Variante binden die Polymere an die Multimerisierungsdomäne des Amyloid beta-Peptids.
Der Begriff "Multimerisierungsdomäne" definiert diejenigen Domänen des Amyloid beta-Peptids, die mit der Interaktion der Amyloid beta-Peptide untereinander zu tun haben. In einer Variante erfüllen die Aminosäuren 10-42 des Amyloid beta-Peptids diese Funktion.

In einer weiteren nicht erfindungsgemäßen Variante weisen die Monomere Sequenzen auf, die sich von den angegebenen Sequenzen um bis zu drei Aminosäuren unterscheiden.
Ferner können als nicht erfindungsgemäße Monomere auch Sequenzen eingesetzt werden, die die oben genannten Sequenzen enthalten.

In einer weiteren nicht erfindungsgemäßen Variante weisen die Monomere Fragmente der oben genannten Sequenzen auf oder weisen homologe Sequenzen zu den oben genannten Sequenzen auf.

"Homologe Sequenzen" oder "Homologe" bedeutet im Sinne der Erfindung, dass eine Aminosäuresequenz eine Identität mit einer der oben genannten Aminosäuresequenz der Monomere von mindestens 50, 55, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 % aufweist. Anstelle des Begriffs "Identität" werden in der vorliegenden Beschreibung die Begriffe "homolog" oder "Homologie" gleichbedeutend verwendet. Die Identität zwischen zwei Nukleinsäuresequenzen oder Polypeptidsequenzen wird durch Vergleich mit Hilfe des Programms BESTFIT basierend auf dem Algorithmus von Smith, T.F. und Waterman, M.S (Adv. Appl. Math. 2: 482-489 (1981)) berechnet unter Einstellung folgender Parameter für Aminosäuren: Gap creation penalty: 8 und Gap extension penalty: 2; und folgender Parameter für Nukleinsäuren: Gap creation penalty: 50 und Gap extension penalty: 3. Bevorzugt wird die Identität zwischen zwei Nukleinsäuresequenzen oder Polypeptidsequenzen durch die Identität der Nukleinsäuresequenz / Polypeptidequenz über die jeweils gesamte Sequenzlänge definiert, wie sie durch Vergleich mit Hilfe des Programms GAP basierend auf dem Algorithmus von Needleman, S.B. und Wunsch, C.D. (J. Mol. Biol. 48: 443-453) unter Einstellung folgender Parameter für Aminosäuren berechnet wird: Gap creation penalty: 8 und Gap extension penalty: 2; und die folgenden Parameter für Nukleinsäuren Gap creation penalty: 50 und Gap extension penalty: 3. Zwei Aminosäuresequenzen sind im Sinne der vorliegenden Erfindung identisch wenn sie die selbe Aminosäuresequenz besitzen.

Unter Homologe sind in einer Variante die entsprechenden retro-inversen Sequenzen der oben genannten Monomere zu verstehen. Mit dem Begriff "retro-inverse Sequenz" wird erfindungsgemäß eine Aminosäuresequenz bezeichnet, die sich aus Aminosäuren in der enantiomeren Form zusammensetzt (invers: Chiralität des alpha-C-Atoms invertiert) und bei der zusätzlich die Sequenzreihenfolge zur ursprünglichen Aminosäuresequenz umgekehrt wurde (retro = rückwärts).

Das Polymer ist aus identischen Monomeren aufgebaut, oder enthält verschiedene Monomere.

In einer nicht erfindungsgemäßen Alternative ist das Polymer aus **jeder beliebige** Kombination aus 2, 3, 4, 5, 6, 7, 8, 9, 10 oder mehr der oben beschriebenen Monomere aufgebaut.

Das Polymer ist ein Dimer aus zwei D3-Monomeren (SEQ ID NO:13).
In einer nicht erfindungsgemäßen Ausführung ist das Polymer ein Dimer aus zwei RD2-Monomeren (RD 2- RD 2: ptlhthnrrrrrptlhthnrrrrr (SEQ ID NO: 76).

Dimere können beispielsweise über chemische Synthese bzw. Peptidsynthese hergestellt werden.

In einer Ausführung der Erfindung sind die Monomere kovalent miteinander verknüpft. In einer weiteren nicht erfindungsgemäßen Ausführung sind die Monomere nicht kovalent miteinander verbunden.

Eine kovalente Verbindung bzw. Verknüpfung der Monomer-Einheiten liegt im Sinne der Erfindung vor, falls die Peptide Kopf an Schwanz linear miteinander verknüpft werden, ohne dass dazwischen Linker oder Linker-Gruppen eingesetzt werden.
Eine nicht kovalente Verknüpfung liegt vor, falls die Monomere über Biotin und Streptavidin, insbesondere Streptavidin-Tetramer miteinander verknüpft sind.

Eine kovalente Verknüpfung kann erreicht werden, indem die Monomer-Einheiten Kopf an Kopf, Schwanz an Schwanz oder Kopf an Schwanz linear gekoppelt werden, jeweils ohne Linker oder mit einer Linker-Gruppe. In einer Alternative ist auch eine Verknüpfung in einem Baum-artigen Gerüst (Dendrimere) auf einem Plattform-Molekül oder einer Kombination dieser Optionen möglich. Hierbei können auch nicht-identische Monomer-Einheiten kombiniert werden.

Das Polymer ist dadurch gekennzeichnet, dass es Amyloid-Beta-Oligomere mit einer Dissoziationskonstante von höchstens 1 mM, bevorzugt 800, 600, 400, 200, 100, 10 µM, besonders bevorzugt 1000, 900, 800, 700, 600, 500, 400, 300, 200, 100, 50 nM, insbesondere bevorzugt, 1000, 900, 800, 700, 600, 500, 400, 300, 200, 100, 50 pM, am meisten bevorzugt höchstens 20 pM bindet.

Das Polymer ist geeignet zur Verwendung in der Medizin.

In einer Ausführung handelt es sich um ein Polymer, welches zur Behandlung von Morbus Alzheimer eingesetzt werden kann. In einer weiteren Ausführung handelt es sich um ein Polymer, welches zur Behandlung von Parkinson, Creutzfeldt Jakob Disease (CJD), Scrapie, bovine spongiforme Enzephalopatie (BSE), oder Diabetes eingesetzt werden kann.

Die aufgebauten Polymere aus Monomeren, die ihrerseits an A-Beta-Oligomeren binden, zeigen deutliche, synergetische Effekte in Bezug auf ihre Selektivität und Affinität zu den A-Beta-Oligomeren im Vergleich zu den Monomeren. Mit anderen Worten: Die erfindungsgemäßen Polymere sind den Monomeren überlegen. Synergetische Effekte im Sinne der vorliegenden Erfindung sind Effekte, die eine höhere Selektivität und Affinität bezüglich der A-Beta-Oligomeren zeigen, insbesondere des K_{D}-Wertes betreffend die Bindung an A-Beta-Oligomere als die Monomere einzeln oder in ihrer Addition.

Unter einem Linker sind ein oder mehrere Moleküle zu verstehen, die über kovalente Bindungen an die Monomere gebunden sind, wobei diese Linker untereinander auch durch kovalente Bindungen verknüpft sein können.
In einer Alternative werden durch die Linker die durch die Monomere vorgegebenen Eigenschaften des Polymers, nämlich die Bindung an A-Beta-Oligomere, nicht verändert.
In einer weiteren Alternative bewirken die Linker eine Veränderung der Eigenschaften des Polymers, die durch die Monomere vorgegeben sind. In einer solchen Ausführung wird die Selektivität und/oder Affinität der Polymere bezüglich der A-Beta-Oligomere verstärkt und/oder die Dissoziationskonstante herabgesetzt. In einer weiteren Ausführung werden die Linker so ausgewählt oder können so angeordnet sein, dass sie die sterische Wirkung der Polymere derart verändern, dass diese selektiv nur an A-Beta-Oligomere einer bestimmten Größe binden.
Eine solche Änderung der sterischen Wirkung der Polymere kann auch durch den Aufbau verzweigter Polymere, durch Dendrimere eines besonderen Aufbaus oder den entsprechenden Aufbau des Polymers mittels Monomere und einem Plattformmolekül oder Kombinationen dieser Optionen erreicht werden.

Weiterer Gegenstand der Offenbarung ist eine Zusammensetzung sowie deren Verwendung zur Bestimmung von Morbus Alzheimer, in der das D-Peptid
a) eine retro-inverse Sequenz des Amyloid beta-Peptids oder Amyloid beta-Peptid-Teilfragmenten enthält und vollständig aus D-Aminosäuren besteht und/oder
b) an die Multimerisierungsdomäne des Amyloid beta-Peptids bindet und/oder
c) die Sequenz SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 und SEQ ID NO:79enthält oder aufweist und vollständig aus D-Aminosäuren besteht und/oder
d) D-Peptide mit der Sequenz SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 und SEQ ID NO:79enthält oder aufweist, wobei die D-Peptide teilweise L-Aminosäuren enthalten und/oder
e) homologe Sequenzen zu SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 und SEQ ID NO:79enthält oder aufweist.

"D-Peptide" bestehen in einer Variante aus einer retro-inversen Sequenz zum Amyloid beta-Peptid oder Amyloid beta-Peptid-Teilfragmenten und vollständig aus D-Aminosäuren.

Ein "Teilfragment" besteht aus 3, 4, 5, 6, 7, 8, 9, 10, 11, 12, 13, 14, 15, 16 oder mehr Aminosäuren homolog zur Aminosäuresequenz des Amyloid beta-Peptids.

In einer weiteren Variante binden die D-Peptide an die Multimerisierungsdomäne des Amyloid beta-Peptids. In einer weiteren Variante weisen die D-Peptide die Sequenz SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 und SEQ ID NO:79 auf und bestehen vollständig aus D-Aminosäuren. In einer weiteren Variante weisen die D-Peptide eine der oben genannten Sequenzen auf, und enthalten teilweise L-Aminosäuren. In einer weiteren Variante weisen die D-Peptide homologe Sequenzen zu den oben genannten Sequenzen auf. Unter "D-Peptid" wird ein Peptid verstanden, welches sich aus Aminosäuren in der D-Form zusammensetzt.

In einer Variante weisen die D-Peptide auch teilweise L-Aminosäuren auf. "Teilweise" L-Aminosäuren bedeutet, dass 1, 2, 3, 4, 5, 6, 7, 8, 9, 10 oder mehr Aminosäuren homolog zur Aminosäuresequenz des aus D-Aminosäuren bestehenden D-Peptids durch jeweils die gleiche Aminosäure in der L-Konformation ersetzt sind.

"D-Peptide" bestehen in einer Variante aus einer retro-inversen Sequenz zum Amyloid beta-Peptid oder Amyloid beta-Peptid-Teilfragmenten und vollständig aus D-Aminosäuren.

Ein "Teilfragment" besteht aus 3, 4, 5, 6, 7, 8, 9, 10, 11, 12, 13, 14, 15, 16 oder mehr Aminosäuren homolog zur Aminosäuresequenz des Amyloid beta-Peptids.

In einer weiteren Variante binden die erfindungsgemäßen D-Peptide an die Multimerisierungsdomäne des Amyloid beta-Peptids. In einer weiteren nicht erfindungsgemäßen Variante weisen die D-Peptide die oben genannten Sequenzen SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 und SEQ ID NO:79auf und bestehen vollständig aus D-Aminosäuren.

In einer anderen Ausführungsform umfasst das Peptid das Sequenzmotiv DB 4: RPRTRLRTHQNR (SEQ ID NO:1) oder aktive Fragmente davon.

In einer anderen Ausführungsform umfasst das Peptid das Sequenzmotiv SHYRHISP (SEQ ID NO:3) oder aktive Fragmente davon.
In einer weiteren Ausführungsform umfasst das Peptid das Sequenzmotiv GISWQQSHHLVA (SEQ ID NO:4) oder aktive Fragmente davon.
In einer weiteren Ausführungsform umfasst das Peptid das Sequenzmotiv PRTRLHTH (SEQ ID NO:5) oder aktive Fragmente davon.

In einer weiteren nicht erfindungsgemäßen Variante wird das Peptid ausgewählt aus der Gruppe, bestehend aus Peptiden mit den D-Aminosäure-Sequenzen : a) QSHYRHISPAQV (SEQ ID NO:6); b) QSHYRHISPDQV (SEQ ID NO:7); c) QSHYRHISPAR (SEQ ID NO:8); d) KSHYRHISPAKV (SEQ ID NO:9); e) RPRTRLHTHRNR (SEQ ID NO:10); i) RPRTRLHTHRTE (SEQ ID NO:11); und g) KPRTRLHTHRNR (SEQ ID NO:12); Bevorzugt sind ferner Sequenzen, die sich von den Sequenzen a) bis g) um bis zu drei Aminosäuren unterscheiden ; sowie Sequenzen, die die Sequenzen a) bis g) sowie Sequenzen, die sich von den Sequenzen a) bis g) um bis zu drei Aminosäuren unterscheiden, umfassen.

In einer weiteren Variante wird das Peptid ausgewählt aus der Gruppe, bestehend aus Peptiden mit den D-Aminosäure-Sequenzen : D3D3: rprtrlhthrnrrprtrlhthrnr (SEQ ID NO:13), aD3nwnD3: rprtrlhthrnrnwnrprtrlhthrnr (SEQ ID NO:14), doppel-D3-freie-Ntermini: (rprtrlhthrnr)2-PEG3 (SEQ ID NO:15), doppel-D3-freie-Ctermini: PEG5-(rprtrlhthrnr)2 (SEQ ID NO:16), oder doppel-D3-freie-Ntermini: (rprtrlhthrnr)2- (SEQ ID NO:63), doppel-D3-freie-Ctermini: (rprtrlhthrnr)2 (SEQ ID NO:64), DB 3: rpitrlrthqnr (SEQ ID NO: 65),RD 2: ptlhthnrrrrr (SEQ ID NO: 66), RD 1: pnhhrrrrrrtl (SEQ ID NO: 67), RD 3: rrptlrhthnrr (SEQ ID NO: 68), D3-delta-hth: rprtrlrnr (SEQ ID NO:69), NT-D3: rprtrl (SEQ ID NO: 70), DB 1: rpitrlhtnrnr (SEQ ID NO: 71), DB 2:rpittlqthqnr (SEQ ID NO: 72), DB 5: rpitrlqtheqr (SEQ ID NO. 74), D3-delta-hth D3-delta-hth: rprtrlrnrrprtrlrnr (SEQ ID NO:75), RD 2- RD 2: ptlhthnrrrrrptlhthnrrrrr (SEQ ID NO: 76), DO 3: sgwhynwqywwk (SEQ ID NO:77), rprtrsgwhynwqywwkrnr (SEQ ID NO:78) und ptlsgwhynwqywwkrrrrr (SEQ ID NO:79). Bevorzugt sind ferner Sequenzen, die sich von den oben genannten Sequenzen um bis zu drei Aminosäuren unterscheiden; sowie Sequenzen, die jede einzelne oder jede beliebisge Kombinatio der oben genannten Sequenzen umfassen.

In einer weiteren Variante weisen die D-Peptide Fragmente der oben genannten Sequenzen auf oder weisen homologe Sequenzen zu den oben genannten Sequenzen auf.

Ferner offenbart sind auch definierte, homogene und stabile Präparationen von Standards zur Quantifizierung von pathogenen Aggregaten oder Oligomeren aus körpereigenen Proteinen, insbesondere zur Behandlung von Blut, Blutprodukten und/oder Organen verwendbar. Einsetzbar sind hier Standards zur Quantifizierung von Oligomeren oder pathogenen Aggregaten, die eine Proteinaggregationserkrankung oder eine amyloide Degeneration oder Proteinvielfaltungserkrankung kennzeichnen. Hierbei wird ein Polymer aus Polypeptidsequenzen aufgebaut, die bezüglich ihrer Sequenz identisch im entsprechenden Teilbereich mit den körpereigenen Proteinen sind oder eine Homologie von mindestens 50 % über den entsprechenden Teilbereich mit den körpereigenen Proteinen aufweisen, die eine Proteinaggregationserkrankung oder eine amyloide Degeneration oder Proteinfehlfaltungserkrankung kennzeichnen, wobei die Polymere nicht aggregieren.

Als Standard im Sinne der vorliegenden Offenbarung wird eine allgemein gültige und akzeptierte, feststehende Bezugsgröße bezeichnet, die zum Vergleichen und Bestimmen von Eigenschaften und/oder Menge dient, insbesondere zur Bestimmung der Größe und Menge von pathogenen Aggregaten aus körpereigenen Proteinen. Der Standard im Sinne der vorliegenden Erfindung kann zum Kalibrieren von Geräten und/oder Messungen verwendet werden.

Im Sinne der vorliegenden Erfindung können unter dem Begriff "Proteinaggregationserkrankung" auch amyloide Degenerationen und Proteinfehlfaltungserkrankungen zusammengefasst werden. Beispiele solcher Krankheiten und die damit verbundenen körpereigenen Proteine sind: A-Beta- und Tau-Protein für AD, alpha-Synuclein für Parkinson, Amylin für Diabetes oder Prion-Protein für Prion-Erkrankungen, zum Beispiel wie die humane Creutzfeldt-Jakob-Krankheit (CJD), die Schafskrankheit Scrapie und die bovine spongiforme Enzephalopatie (BSE).

Unter dem Begriff "entsprechender Teilbereich" körpereigener Proteine ist jene Peptidsequenz zu verstehen, die gemäß den erfindungsgemäßen Definitionen eine identische oder mit der angegebenen Prozentzahl homologe Peptidsequenz eines Monomers, aus dem die erfindungsgemäßen Standards aufgebaut werden, aufweist.

Wesentlich für die Standards ist, dass die Standards nicht aggregieren, bevorzugt durch die Verwendung von monomeren Sequenzen, die nicht aggregieren, da der "entsprechender Teilbereich" körpereigener Proteine für die Aggregation nicht verantwortlich ist, oder die durch Blockierung der für die Aggregation verantwortlichen Gruppen nicht aggregieren.

Aggregate im Sinne der vorliegenden Offenbarung sind
- Partikel die aus mehreren, bevorzugt gleichen Bausteinen bestehen, die nicht kovalent miteinander verbunden sind und/oder
- nicht-kovalente Zusammenlagerungen mehrerer Monomere.

In einer Ausführung besitzen die Standards eine genau definierte Anzahl von Epitopen, die kovalent miteinander verknüpft sind (unmittelbar oder über Aminosäuren, Spacer und/oder funktionelle Gruppen) für die Bindung der entsprechenden Sonden.
Sonden im Sinne der Offenbarung werden ausgewählt aus der Gruppe bestehend aus: Antikörper, Nanobody und Affibody.

Die Zahl der Epitope wird dadurch bestimmt, dass eine Polypeptid-Sequenz verwendet wird, die bezüglich ihrer Sequenz identisch mit jenem Teilbereich der körpereigenen Proteine ist, die ein Epitop bildet bzw. eine Homologie von mindestens 50 % mit diesem Teilbereich aufweist, und dabei die biologische Aktivität des Epitopes besitzt.

In einer weiteren Ausführung handelt es sich bei den Epitopen um Epitope des A-Beta-Peptids ausgewählt aus den Teilbereichen A-Beta 1-11, A-Beta 3-11 oder pyroGluA-Beta 3-11, zum Beispiel des humanen N-terminale Epitops (mit folgender Sequenz: DAEFRHDSGYE (1-11) (SEQ ID NO:17)).

Das Standardmolekül ist ein Polymer aus den oben definierten Polypeptid-Sequenzen. Unter Oligomer im Sinne der Erfindung ist ein Polymer aus 2, 3, 4, 5, 6, 7, 8, 9, 10, 11, 12, 13, 14, 15, 16, 17, 18, 19 oder 20 Monomeren (unter Monomer ist die o.g. Polypeptid-Sequenz zu verstehen) gebildet, oder Vielfache davon, bevorzugt 2 - 16, 4-16, 8-16, besonders bevorzugt 8 oder 16, oder Vielfache davon.

In einer Alternative sind die Standards wasserlöslich.

In einer Alternative sind die Standards aus gleichen Polypeptid-Sequenzen aufgebaut.
In einer weiteren Alternative sind die Standards aus unterschiedlichen Polypeptid-Sequenzen aufgebaut.

In einer Alternative werden solche oben definierte Polypeptid-Sequenzen in einer linearen-Konformation aneinandergereiht.
In einer Alternative werden solche oben definierte Polypeptid-Sequenzen zu einem verzweigten, erfindungsgemäßen Oligomer aneinandergereiht.

In einer Alternative werden solche oben definierte Polypeptid-Sequenzen zu einem cross-linked, Oligomer aneinandergereiht.
Verzweigte oder cross-linked, Oligomere könne durch Verknüpfung einzelner Bausteine mittels Lysin oder mittels Click-Chemie hergestellt werden.

Die Offenbarung betrifft in einer Alternative ein Standardmolekül, enthaltend oder aufgebaut aus Kopien des aminoterminalen Teils des A-Beta-Peptids, ausgewählt aus den Teilbereichen A-Beta 1-11, A-Beta 3-11, oder pyroGluA-Beta 3-11, zum Beispiel des humanen N-terminale Epitops (mit folgender Sequenz: DAEFRHDSGYE (1-11) (SEQ ID NO:17)).

Die Vervielfältigung der Epitope durch funktionelle Gruppen kann vor oder nach der Synthese der einzelnen Bausteine durchgeführt werden. Charakteristisch für die Standards ist die kovalente Verknüpfung der Polypeptid-Sequenzen.

Die einzusetzenden Polypeptid-Sequenzen können identisch mit der Sequenz des A-Beta-Volllängen-Peptids sein oder zeigen eine Homologie von 50, 55, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 % mit der Sequenz des A-Beta-Volllängen-Peptid.

Alternativ werden auch zum Aufbau der Standard-Moleküle Polypeptid-Sequenzen eingesetzt, die identisch mit einem Teilbereich des A-Beta-Volllängen-Peptids sind, bzw. eine Homologie von 50, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 % mit einem Teilbereich des A-Beta-Volllängen-Peptids zeigen.
Wesentlich für die eingesetzten Sequenzen ist ihre Eigenschaft nicht (oder nur gemäß den Bedingungen kontrolliert) zu aggregieren und/oder ihre die Aktivität als Epitop.

In einer weiteren Ausführung sind die Standards als Dendrimere aufgebaut. Die Dendrimere sind aus den oben beschriebenen zu verwendenden Polypeptid-Sequenzen aufgebaut und können ein zentrales Gerüstmolekül enthalten.

Die Dendrimere enthalten in einer Variante Polypeptid-Sequenzen, die eine Sequenz besitzen, die identisch mit einem Teilbereich des A-Beta-Peptids ist, oder eine mindestens 50 %ige Homologie zu dem entsprechenden Teilbereich zeigt.

Unter dem Begriff mindestens 50 %ige Homologie auch eine höhere Homologie zu verstehen, ausgewählt aus der Gruppe bestehend aus 50, 55, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 % zu verstehen.

Standards, vorteilhaft mit höherer Löslichkeit im Wässrigen als pathogene Aggregate oder Oligomere aus körpereigenen Proteinen, sind in einer Ausführung aus Polypeptid-Sequenzen gebildet, die identisch mit dem N-terminalen Bereich des A-Beta-Peptids sind oder mindestens 50 %ige Homologie dazu aufweisen. Gemäß der Offenbarung ist unter dem N-terminalen Bereich von einem A-Beta-Polypeptid die Aminosäuresequenz A-Beta 1 - 8, A-Beta 1 - 11, A-Beta 1 - 16, A-Beta 3-11 oder pyroGluA-Beta 3-11 zu verstehen.

Ein einsetzbares Standardmolekül kann Epitope für mindestens 2, 3, 4, 5, 6, 7, 8, 9, 10 oder mehr verschiedene Sonde enthalten.

Epitope charakteristisch für verschiedene Sonden können in die Standards dadurch eingebaut werden, dass Polypeptid-Sequenzen verwendet werden, die identisch mit unterschiedlichen Bereichen des A-Beta-Peptids sind, bzw. mindestens eine 50 %ige Homologie dazu aufweisen, aber die Aktivität des entsprechenden Epitops besitzen.
In einer Ausführung werden hierzu Polypeptidsequenzen eingesetzt, die identisch oder eine 50 %ige Homologie mit dem N-terminalen Bereich des A-Beta-Polypeptids aufweisen, sowie Polypeptid-Sequenzen, die identisch bzw. mindestens eine 50 %ige Homologie mit dem C-Terminus des A-Beta-Polypeptids aufweisen.

In einer Ausführung der enthalten die Standardmoleküle sog. Spacer.
Unter einem Spacer ist ein Molekül zu verstehen, das über kovalente Bindungen in das Standardmolekül eingebaut ist, und bestimmte physikalische und/oder chemische Eigenschaften besitzt, durch welche die Eigenschaften des Standardmoleküls verändert werden. In einer Ausführung der Standards werden hydrophile oder hydrophobe, bevorzugt hydrophile Spacer, eingesetzt. Hydrophile Spacer werden ausgewählt aus der Gruppe der Moleküle, gebildet aus Polyethylenglycol, Zucker, Glycerin, Poly-L-Lysin oder beta-Alanin.

Die einsetzbaren Standards enthalten in einer Alternative (weitere) funktionelle Gruppen. Unter funktionellen Gruppen sind Moleküle zu verstehen, die kovalent an die Standardmoleküle gebunden sind. In einer Variante enthalten die funktionellen Gruppen Biotin-Gruppen. Dadurch wird eine starke kovalente Bindung an Streptavidin ermöglicht. Standardmoleküle enthaltend Biotin-Gruppen können so an Moleküle, enthaltend Streptavidin-Gruppen, gebunden werden. Falls die einsetzbaren Standardmoleküle Biotin und/oder Streptavidin-Gruppen enthalten, können so größere Standards zusammengebaut werden oder mehrere, ggf. unterschiedliche Standardmoleküle, an ein Gerüst gebunden werden.

In einer weiteren Alternative enthalten die Standardmoleküle Farbstoffe zur spektralphotometrischen Bestimmung und/oder aromatische Aminosäuren. Aromatische Aminosäuren sind z.B. Tryptophane, Tyrosin, Phenylalanin oder Histidin, bzw. ausgewählt aus dieser Gruppe. Durch den Einbau von Tryptophan wird eine spektralphotometische Bestimmung der Konzentration von Standards in Lösung ermöglicht.

Weiterer Gegenstand sind ist der Einsatz von Dendrimeren enthaltenden Polypeptiden, die bezüglich ihrer Sequenz identisch im entsprechenden Teilbereich mit den körpereigenen Proteinen sind oder eine Homologie von mindestens 50 % über den entsprechenden Teilbereich mit den körpereigenen Proteinen aufweisen, die eine Proteinaggregationserkrankung kennzeichnen.

Die Dendrimere können jede der oben beschriebenen Merkmale der Standards oder jede beliebige Kombination davon enthalten.

In einer Alternative handelt es sich Dendrimere enthaltend eine genau definierte Anzahl von Epitopen für die kovalente Bindung von Sonden besitzt,
Dendrimer enthaltend Epitope des A-beta-Peptids.

Zur Untersuchung von Blut, Blutprodukten und/oder Organen auf Amyloid-Beta-Oligomere kann ein Standard eingesetzt werden, zur Quantifizierung von pathogenen Aggregaten oder Oligomeren aus körpereigenen Proteinen, die eine Proteinaggregationserkrankung oder eine amyloide Degeneration oder Proteinfehlfaltungserkrankung kennzeichnen, dadurch gekennzeichnet, dass ein Polymer aus Polypeptidsequenzen aufgebaut wird, die bezüglich ihrer Sequenz identisch im entsprechenden Teilbereich mit den körpereigenen Proteinen sind oder eine Homologie von mindestens 50 % über den entsprechenden Teilbereich mit jenen körpereigenen Proteinen aufweisen, die eine Proteinaggregationserkrankung oder eine amyloide Degeneration oder Proteinfehlfaltungserkrankung kennzeichnen, wobei die Polymere nicht aggregieren.

Der Standard kann für die Bindung von Sonden eine genau definierte Anzahl von Epitopen besitzen, die kovalent miteinander verknüpft sind.

Der Standard kann auch Epitope des A-beta-Peptids enthalten und/oder die erfindungsgemäßen Sequenzen.

Zur Untersuchung von Blut, Blutprodukten und/oder Organen auf Amyloid-Beta-Oligomere kann auch folgendes Verfahren zur selektiven Quantifizierung von A-Beta-Aggregaten eingesetzt werden:
Verfahren umfassend die Immobilisierung von A-Beta-Fängermolekülen auf einem Substrat, Auftragen der zu untersuchenden Probe auf das Substrat, Hinzufügen von für die Detektion gekennzeichneten Sonden, die durch spezifische Bindung an A-Beta-Aggregate diese markieren und Detektion der markierten Aggregate.

Dieses Verfahren zur selektiven Quantifizierung und/oder Charakterisierung von A-Beta-Aggregaten umfasst folgende Schritte:
a) Auftragen der zu untersuchenden Probe auf das Substrat,
b) Hinzufügen von für die Detektion gekennzeichneten Sonden, die durch spezifische Bindung an A-Beta-Aggregate diese markieren und
c) Detektion der markierten Aggregate, wobei
Schritt b) vor Schritt a) durchgeführt werden kann.

Es werden auch Standards zur Verfügung gestellt, die eine exakte und quantitative Bestimmung von pathogenen Aggregaten oder Oligomeren aus körpereigenen Proteinen möglich machen. Die Standards sollen als interne oder externe Standards einsetzbar sein.

Ferner können genau definierte, homogene und stabile Präparationen von Standards zur Quantifizierung von pathogenen Aggregaten oder Oligomeren aus körpereigenen Proteinen verwendet werden.

Standards zur Quantifizierung von Oligomeren oder pathogenen Aggregaten, die eine Proteinaggregationserkrankung oder eine amyloide Degeneration oder Proteinfehlfaltungserkrankung kennzeichnen, sind dadurch gekennzeichnet, dass ein Polymer aus Polypeptidsequenzen aufgebaut wird, die bezüglich ihrer Sequenz identisch im entsprechenden Teilbereich mit den körpereigenen Proteinen sind oder eine Homologie von mindestens 50 % über den entsprechenden Teilbereich mit den körpereigenen Proteinen aufweisen, die eine Proteinaggregationserkrankung oder eine amyloide Degeneration oder Proteinfehlfaltungserkrankung kennzeichnen, wobei die Polymere nicht aggregieren.

Der Standard kann dadurch gekennzeichnet sein, dass dieser eine genau definierte Anzahl von Epitopen, die kovalent miteinander verknüpft sind, für die Bindung von Sonden besitzt.

Der Standard kann ferner dadurch gekennzeichnet sein, dass er Epitope des A-beta-Peptids und/oder die erfindungsgemäßen Sequenzen enthält.
Die Standards werden als Fängermoleküle eingesetzt. In einer Alternative werden die Standards zur Entfernung und/oder Enttoxifizierung von Amyloid-Beta-Oligomere in Blut, Blutprodukten und/oder Organen verwendet.

Ferner offenbart ist ein Kit, welches Standard und/oder Sequenzen umfasst. Die Verbindungen und/oder Komponenten des Kits können in Behältern gegebenenfalls mit/in Puffern und/oder Lösung, verpackt sein. Alternativ können einige Komponenten in demselben Behälter verpackt sein. Zusätzlich dazu oder alternativ dazu könnten eine oder mehrere der Komponenten an einem festen Träger, wie z.B. einer Glasplatte, einem Chip oder einer Nylonmembran oder an die Vertiefung einer Mikrotiterplatte, absorbiert sein. Ferner kann das Kit Anweisungen für den Gebrauch des Kits für eine Beliebige der Ausführungsformen enthalten.

Gegenstand der vorliegenden Erfindung ist auch ein Kit zur selektiven Quantifizierung von A-Beta-Aggregaten gemäß dem oben beschriebenen Verfahren. Ein solches Kit kann ein oder mehrere der folgenden Komponenten enthalten:
- Substrat aus Glas das mit einem hydrophoben Stoff beschichtet ist;
- Standard;
- Fängermolekül;
- Sonde;
- Substrat mit Fängermolekül;
- Lösungen;
- Puffer.

Die Verbindungen und/oder Komponenten des Kits der vorliegenden Erfindung können in Behältern gegebenenfalls mit/in Puffern und/oder Lösung, verpackt sein. Alternativ können einige Komponenten in demselben Behälter verpackt sein. Zusätzlich dazu oder alternativ dazu könnten eine oder mehrere der Komponenten an einem festen Träger, wie z.B. einer Glasplatte, einem Chip oder einer Nylonmembran oder an die Vertiefung einer Mikrotiterplatte, absorbiert sein. Ferner kann das Kit Anweisungen für den Gebrauch des Kits für eine Beliebige der Ausführungsformen enthalten.
In einer weiteren Variante des Kits sind auf dem Substrat die oben beschriebenen Fängermoleküle immobilisiert. Zusätzlich kann das KIT Lösungen und/oder Puffer enthalten. Zum Schutz der Dextranoberfläche und/oder der darauf immobilisierten Fängermoleküle können diese mit einer Lösung oder einem Puffer überschichtet werden.

Das Kit kann auch mindestens einen Standard oder mindestens ein Dendrimer zur Quantifizierung von pathogenen Aggregaten oder Oligomeren aus körpereigenen Proteinen, die eine Proteinaggregationserkrankung enthalten.

Polymere, D-Peptide, Antikörper, und/oder Verbindungen die erfindungsgemäß in einem Verfahren zur Behandlung (in vitro) von Blut, Blutprodukten und/oder Organen zur Entfernung und/oder Entoxifizierung von Amyloid-Beta-Oligomeren einsetzbar sind, sind nachfolgend dargestellt:
D3D3: rprtrlhthrnrrprtrlhthrnr (SEQ ID NO:13)

Weitere Polymere, D-Peptide, Antikörper, und/oder Verbindungen die in einem nicht erfindungegemäßen Verfahren zur Behandlung (in vitro) von Blut, Blutprodukten und/oder Organen zur Entfernung und/oder Entoxifizierung von Amyloid-Beta-Oligomeren einsetzbar sind, sind nachfolgend dargestellt:
DB 4: rprtrlrthqnr (SEQ ID NO:1)
D3nwnD3: rprtrlhthrnrnwnrprtrlhthrnr (SEQ ID NO:14)
doppel-D3-freie-Ntermini: (rprtrlhthrnr)₂-PEG3 (SEQ ID NO:15)
doppel-D3-freie-Ctermini: PEG5-(rprtrlhthrnr)₂ (SEQ ID NO:16)
kqhhveygsdhrfead (SEQ ID NO:2)
shyrhisp (SEQ ID NO:3)
giswqqshhlva (SEQ ID NO:4)
prtrlhth (SEQ ID NO:5)
qshyrhispaqv (SEQ ID NO:6)
qshyrhispdqv (SEQ ID NO:7)
qshyrhispar (SEQ ID NO:8)
kshyrhispakv (SEQ ID NO:9)
rprtrlhthrnr (SEQ ID NO:10)
rprtrlhthrte (SEQ ID NO:11)
kprtrlhthrnr (SEQ ID NO:12)
daefrhdsgye (SEQ ID NO:17)
hhghspnvsqvr (SEQ ID NO:18)
gsfstqvgslhr (SEQ ID NO:19)
htgtqsyvprl (SEQ ID NO:20)
tlayaraymvap (SEQ ID NO:21)
tlayaraymvap (SEQ ID NO:22)
atpqndlktfph (SEQ ID NO:23)
tqpetdllrvqf (SEQ ID NO:24)
citwpptglty (SEQ ID NO:25)
tfletgpiyadg (SEQ ID NO:26)
lvppthrhwpvt (SEQ ID NO:27)
appgnwrnylmp (SEQ ID NO:28)
dnysnyvpgtkp (SEQ ID NO:29)
svsvgmkpsprp (SEQ ID NO:30)
slpnpfsvssfg (SEQ ID NO:31)
yvhnpyhlpnpp (SEQ ID NO:32)
crrlhtyigpvt (SEQ ID NO:33)
gatmkkmddhtv (SEQ ID NO:34)
lgktqklsdahs (SEQ ID NO:35)
ddqarpymaygp (SEQ ID NO:36)
gdtwvnmvsmvh (SEQ ID NO:37)
gytwvnmvsmvh (SEQ ID NO:38)
wtntvarlatpy (SEQ ID NO:39)
qtqalyhsrqvh (SEQ ID NO:40)
nsqtqtlhlfph (SEQ ID NO:41)
hntsanilhssh (SEQ ID NO:42)
shinptsfwpap (SEQ ID NO:43)
tfsnplymwprp (SEQ ID NO:44)
gpspfnpqptpv (SEQ ID NO:45)
fsdhksptpppr (SEQ ID NO:46)
stsvyppppsaw (SEQ ID NO:47)
yglptqansmql (SEQ ID NO:48)
hnrtdntyirpt (SEQ ID NO:49)
lqqplgnnrpns (SEQ ID NO:50)
kpedsaaypqnr (SEQ ID NO:51)
rpedsvitktqnt (SEQ ID NO:52)
raadsgctptkh (SEQ ID NO:53)
rprtrlhthrnt (SEQ ID NO:54)
rprtrlhthtnv (SEQ ID NO:55)
rprtrlhthtnr (SEQ ID NO:56)
rprtrlhthrkq (SEQ ID NO:57)
rprtrlhtlrnr (SEQ ID NO:58)
rrrsplhthrnr (SEQ ID NO:59)
lrsprqrripri (SEQ ID NO:60)
rkrqlrmttprp (SEQ ID NO:61)
shyrhispaqk (SEQ ID NO:62)
doppel-D3-freie-Ntermini: (rprtrlhthrnr)2- (SEQ ID NO:63)
doppel-D3-freie-Ctermini: (rprtrlhthrnr)2 (SEQ ID NO:64)
DB 3: rpitrlrthqnr (SEQ ID NO: 65),
RD 2: ptlhthnrrrrr (SEQ ID NO: 66)
RD 1: pnhhrrrrrrtl (SEQ ID NO: 67)
RD 3: rrptlrhthnrr (SEQ ID NO: 68)
D3-delta-hth: rprtrlrnr (SEQ ID NO:69)
NT-D3: rprtrl (SEQ ID NO: 70)
DB 1: rpitrlhtnrnr (SEQ ID NO: 71),
DB 2:rpittlqthqnr (SEQ ID NO: 72),
D3: rprtrlhthrnr (SEQ ID NO: 73 ()
DB 5: rpitrlqtheqr (SEQ ID NO. 74)
D3-delta-hth D3-delta-hth: rprtrlrnrrprtrlrnr (SEQ ID NO:75)
RD 2- RD 2: ptlhthnrrrrrptlhthnrrrrr (SEQ ID NO: 76)
DO 3: sgwhynwqywwk (SEQ ID NO:77)
rprtrsgwhynwqywwkrnr (SEQ ID NO:78)
ptlsgwhynwqywwkrrrrr (SEQ ID NO:79)

Antikörper, die in einem Verfahren zur Behandlung (in vitro) von Blut, Blutprodukten und/oder Organen zur Entfernung und/oder Entoxifizierung von Amyloid-Beta-Oligomeren einsetzbar sind, sind nachfolgend definiert:
Antikörper, die
a) an eine retro-inverse Sequenz des Amyloid beta-Peptids oder Amyloid beta-Peptid-Teilfragmenten binden und/oder
b) an die Multimerisierungsdomäne des Amyloid beta-Peptids binden und auch an das Amyloid beta-Peptid und/oder
c) an eine der oben genannten Sequenzen ausgewählt aus der Gruppe:
   SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 und SEQ ID NO:79 oder homologe Sequenzen davon;
   binden.

Hybrid-Verbindungen, die in einem Verfahren zur Behandlung (in vitro) von Blut, Blutprodukten und/oder Organen zur Entfernung und/oder Entoxifizierung von Amyloid-Beta-Oligomeren einsetzbar sind, sind nachfolgend definiert:
Hybrid-Verbindung der Formel A-B wobei A ein Aminopyrazol oder ein Derivat davon ist und B ein Peptid und A und B unmittelbar oder mittels eines Linkers kovalent miteinander verbunden sind.

Hybrid-Verbindung, dadurch gekennzeichnet, dass B ein D-Peptid ist, dadurch gekennzeichnet, dass das D-Peptid ausgewählt ist aus den oben genannten Sequenzen .

Hybrid-Verbindung der Formel A-B, dadurch gekennzeichnet, dass
A ausgewählt ist aus der Gruppe der Verbindungen bestehend aus:
   3-Aminopyrazol-5-carbonsäure, Derivate davon mit ersetzter heterocyclischer CH-Gruppe gegen -CR- oder -N-, -O-, -S-, 3-Aminopyrazol-5-carbonsäure-Dimer sowie -Trimer oder -Tetramer.
B ausgewählt ist aus der Gruppe der Verbindungen bestehend aus:
   D3-Peptid, SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 und SEQ ID NO:79;
   der Linker ausgewählt ist aus der Gruppe der Verbindungen bestehend aus:
   kein Linker, GABA, TEG, TEG-Dimer, PEG, alpha-Aminosäuren, alpha-Amino-omegacarbonsäure.

Durch das erfindungsgemäße Verfahren werden pathogene Partikel, Amyloid-Beta-Oligomere entfent und/oder enttoxifiert, also in eine unschädliche Form überführt oder vernichtet. Somit wird eine weiter Infektion durch Verbleib und Vermehrung der pathogenen (amyloidogenen) Partikel vermieden. Dabei treten synergetische Effekte auf, ausgelöst durch die erfindungsgemäß einzusetzenden Verbindungen. So führen insbesondere die Ko-Aggregate aus Amyloid-Beta-Peptiden (-Oligomeren) und den erfindungsgemäß einzusetzenden Verbindungen als unschädliche Form zu einer späteren Verhinderung oder Reduzierung weiteren oder neuen Bildung von A-Beta-Aggeregaten. Das erfindungsgemäße Verfahren ist also sicherer als die aus dem Stand der Technik bekannten Verfahrten, da keine absolut restlose Entfernung der pathogegen, toxischen und/oder infektiösen Partikeln erfolgen muß. Es findet ein Enttoxifizierung durch Überführung in unschädliche Formen statt, die soger eine spätere Infektion, also die Bildung von A-Beta-Aggeregaten, verhindern oder reduzieren kann.

### Beispiele:

### 1. ThT-Seedingassay: Grundlagen

Amyloidogene Peptide besitzen die Fähigkeit, amyloide Fibrillen zu bilden. Dies kann mit einer gewissen Wahrscheinlichkeit spontan geschehen. Sind keine amyloiden "Keime" vorhanden, kann es einige Zeit dauern, bis die ersten Amyloid-Fibrillen gebildet werden, deren Bildung und Vermehrung mit Hilfe der Fluoreszenz von Thioflavin T (ThT) quantitativ verfolgt werden kann. ThT interagiert mit A-Beta Fibrillen und der Fibrillen-Farbstoff-Komplex zeigt eine erhöhte Fluoreszenz (λem: 450 nm, λex: 490 nm). Die Zeit bis das ThT-Signal zu steigen beginnt, wird "lag phase" genannt. Diese "lag phase" kann vermieden oder stark verkürzt werden, wenn man amyloide "Keime", auch "seeds" genannt, zum Aggregationsansatz hinzu gibt. Ein bekanntes Beispiel ist das Hinzugeben von Prion-haltigem Hirnmaterial zu einer Lösung von monomerem rekombinanten Prionprotein, das daraufhin mit einer deutlich verkürzten "lag phase" ThT-positive Fibrillen bildet. Ein weiteres Beispiel besteht im Hinzufügen einer kleinen Menge von Abeta-Amyloid-Fibrillen zu einer Lösung von nicht-aggregiertem A-Beta-Peptid (Abeta). Auch dabei wird die "lag phase" zur Bildung ThT-positiver Abeta-Fibrillen deutlich verkürzt. Dadurch erlaubt es dieser Test (genannt "ThT-Seedingassay"), beliebige Substanzen oder Substanzgemische auf ihren Gehalt an Keim-fähigen Amyloiden zu untersuchen. Enthält das dem Aggregationsansatz hinzugegebene Substanzgemisch "seed"-fähige Amyloide, wirkt sich dies im Fehlen oder Verkürzen der "lag phase" aus. Diese in vitro-Eigenschaft wird oft analog zur Prion-ähnlichen Infektiosität oder Übertragbarkeit in vivo betrachtet. Beim ersten Kontroll-Versuch wurde untersucht, ob vorgeformte Abeta-Aggregate als Aggregationskeime tatsächlich die "lag phase" der Aggregation von frisch gelöstem Abeta verkürzen. Dazu wurde die ThT-Fluoreszenz von frisch gelöstem Abeta in Abwesenheit und Anwesenheit von vorgeformten Abeta-Aggregaten verfolgt. Anschließend wurden Abeta-Aggregate präpariert, die aus einer Mischung aus Abeta(1-42) und einer Inhibitor-Substanz (in diesem Beispiel je eines der D-Peptide D3, DB1, DB2, DB3, DB4, DB5) gebildet wurden, die die Bildung von amyloiden Fibrillen stören soll. Die so gebildeten Abeta-Inhibitor-Koaggregate wurden genau wie die amyloiden Abeta-Aggregate zu einem ThT-Seedingassay gegeben, um das verbliebene amyloide Potential dieser Koaggregate zu bestimmen.

### 2. ThT-Seedingassay: Experimentelle Details

Es wurde 20 µM A-Beta-Peptid (1-42) zusammen mit einer der Inhibitor-Substabnzen (20 µM) für 7 Tage bei 37 °C in 10 mM NaPi pH 7.4 vorinkubiert. Anschließend wurde die Probe zentrifugiert (20 min, 16.100 x g), das Aggregat-Pelett 3x gewaschen und in 10 mM NaPi pH 7.4 resuspendiert. In Analoger weise wurden Aß-Fibrillen ohne Inhibitor als positiv-Kontrolle hergestellt. Direkt vor dem eigentlichen ThT-Seedingassay wurde frisches Aß (20 µM in 10 mM NaPi pH 7.4) mit den resuspendierten Aggregationskeimen, die in Anwesenheit oder Abwesenheit der Inhibitor-Substanzen entstanden waren, vermischt (80:20 Volumenanteile) und 10 µM ThT dazugegeben. Als Referenz diente frische Aß-Lösung, die mit Pufferlösung ohne Aggregationskeimen vermischt wurde (80:20 Volumenanteile) und 10 µM ThT enthielt. 50 µl der jeweiligen Reaktionslösung wurden in eine Vertiefung einer schwarzen 384-Well-Mikrotiterplatte pipettiert. Die ThT-Fluoreszenz wurde alle 30 min über 20 h bei einer Anregungswellenlänge von 440 nm und einer Emissionswellenlänge von 490 nm gemessen. Für die Auswertung wurde die Fluoresnzenzintensität durch Subtraktion der 20% zugesetzten Aggregationskeime korrigiert und der Mittelwert berechnet. Es wurde eine achtfache Bestimmung durchgeführt.

### 3. Durchführung:

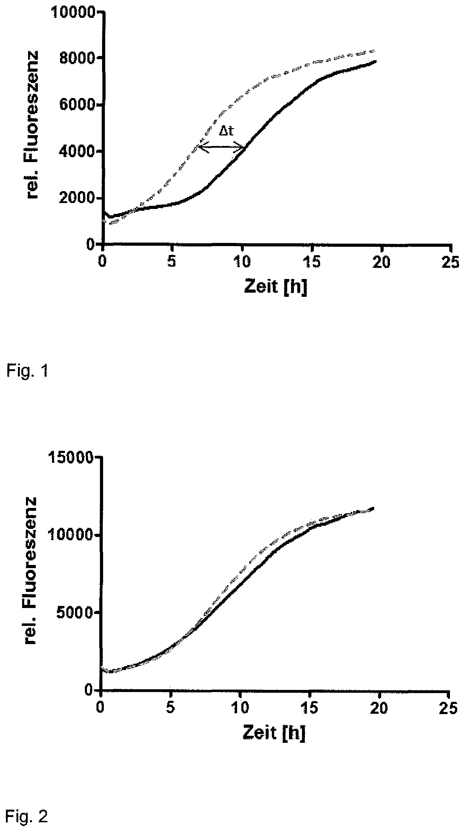

Fig. 1 zeigt den Zeitverlauf der ThT-Fluoreszenz in Abwesenheit (durchgezogene Linie) und Anwesenheit von vorgeformten Abeta-Aggregaten, die ohne Zusatz von Inhibitor-Substanzen entstanden sind.
   Es ist klar zu erkennen, dass diese Aggregate die Aggregation von frischem Abeta deutlich beschleunigen (Δt von etwa 4 h), also eindeutig einen Seeding-Effekt zeigen.
Fig. 2 zeigt den Zeitverlauf der ThT-Fluoreszenz in Abwesenheit (durchgezogene Linie) und Anwesenheit von vorgeformten Abeta-Aggregaten, die vor dem Versuchsstart unter Zusatz der Inhibitor-Substanz D3 entstanden sind.
   Es ist klar zu erkennen, dass diese Aggregate die Aggregation von frischem Abeta nicht beschleunigen können, also eindeutig keinen Seeding-Effekt zeigen.

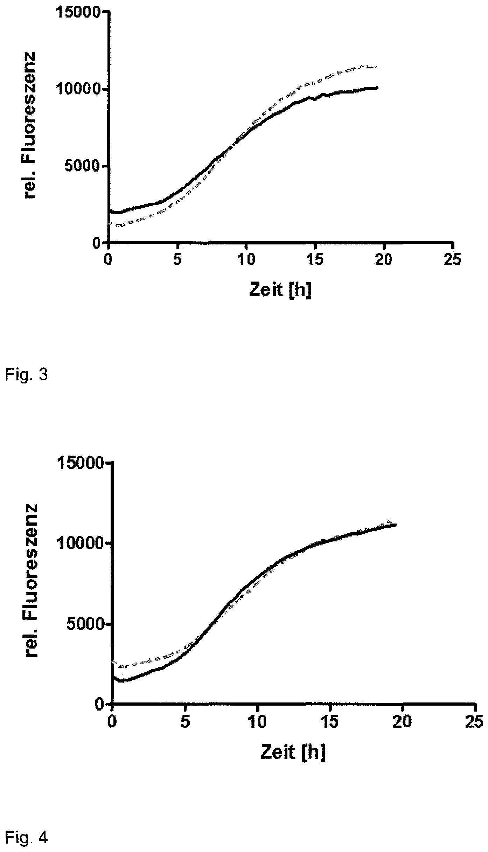

Fig. 3 zeigt den Zeitverlauf der ThT-Fluoreszenz in Abwesenheit (durchgezogene Linie) und Anwesenheit von vorgeformten Abeta-Aggregaten, die vor dem Versuchsstart unter Zusatz der Inhibitor-Substanz DB1 entstanden sind.
   Es ist klar zu erkennen, dass diese Aggregate die Aggregation von frischem Abeta nicht beschleunigen können, also eindeutig keinen Seeding-Effekt zeigen.
Fig. 4 zeigt den Zeitverlauf der ThT-Fluoreszenz in Abwesenheit (durchgezogene Linie) und Anwesenheit von vorgeformten Abeta-Aggregaten, die vor dem Versuchsstart unter Zusatz der Inhibitor-Substanz DB2 entstanden sind.
   Es ist klar zu erkennen, dass diese Aggregate die Aggregation von frischem Abeta nicht beschleunigen können, also eindeutig keinen Seeding-Effekt zeigen.

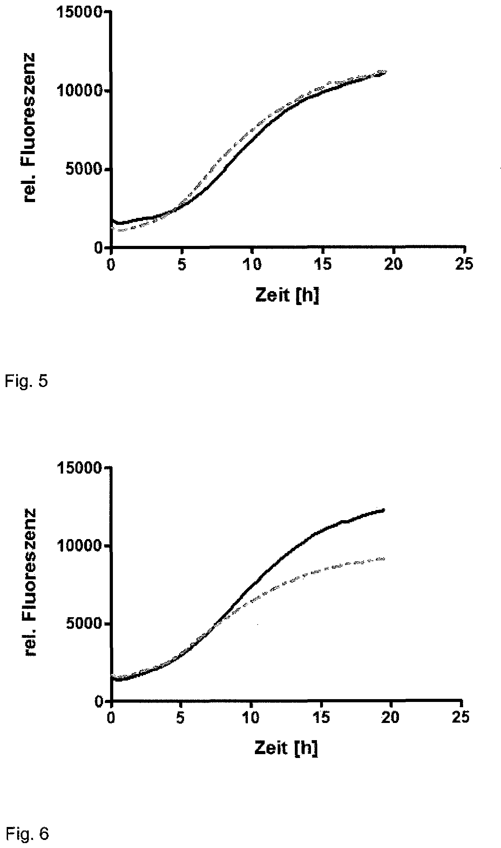

Fig. 5 zeigt den Zeitverlauf der ThT-Fluoreszenz in Abwesenheit (durchgezogene Linie) und Anwesenheit von vorgeformten Abeta-Aggregaten, die vor dem Versuchsstart unter Zusatz der Inhibitor-Substanz DB3 entstanden sind.
   Es ist klar zu erkennen, dass diese Aggregate die Aggregation von frischem Abeta nicht beschleunigen können, also eindeutig keinen Seeding-Effekt zeigen.
Fig. 6 zeigt den Zeitverlauf der ThT-Fluoreszenz in Abwesenheit (durchgezogene Linie) und Anwesenheit von vorgeformten Abeta-Aggregaten, die vor dem Versuchsstart unter Zusatz der Inhibitor-Substanz DB4 entstanden sind.
   Es ist klar zu erkennen, dass diese Aggregate die Aggregation von frischem Abeta nicht beschleunigen können, also eindeutig keinen Seeding-Effekt zeigen. Darüber hinaus scheinen die DB4-Abeta-Koaggregate sogar die Bildung von ThT-positiven Abeta-Aggregaten in der späteren Phase zu reduzieren.

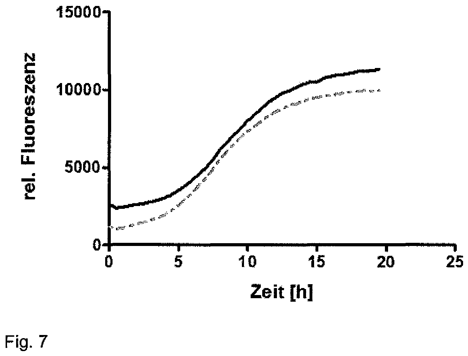

Fig. 7 zeigt den Zeitverlauf der ThT-Fluoreszenz in Abwesenheit (durchgezogene Linie) und Anwesenheit von vorgeformten Abeta-Aggregaten, die vor dem Versuchsstart unter Zusatz der Inhibitor-Substanz DB5 entstanden sind.

Es ist zu erkennen, dass diese Aggregate die Aggregation von frischem Abeta nicht beschleunigen können, also eindeutig keinen Seeding-Effekt zeigen.

### 4. Zusammenfassung der Ergebnisse:

Alle getesteten D-Peptide bildeten mit Abeta Aggregate, die nicht mehr in der Lage waren, die "lag phase" der Aggregation von frisch gelöstem Abeta zu verkürzen. Diese Ko-Aggregate sind also nicht amyloidogen.

## Patentansprüche

1. Verfahren zur Behandlung außerhalb des menschlichen oder tierischen Körpers (in vitro, ex vivo) von Blut, Blutprodukten und/oder Organen indem Amyloid-Beta-Oligomere entfernt und/oder enttoxifiziert werden **dadurch gekennzeichnet, dass** die D-enantiomere Verbindung rprtrlhthrnrrprtrlhthrnr (D3D3, SEQ ID NO:13) eingesetzt wird, die an Amyloid-Beta-Oligomere bindet.

2. Verfahren nach Anspruch 1 **dadurch gekennzeichnet, dass** vor der Behandlung das Blut, die Blutprodukte oder die Organe auf Amyloid-Beta-Oligomere untersucht werden.

3. Verfahren nach einem der vorangehenden Ansprüche **dadurch gekennzeichnet, dass** die Verbindungen aus Anspruch 1 als Fängermoleküle auf einem Träger angeordnet sind, über das eine das Blut, die Blutprodukte und/oder Organ enthaltende Probe geleitet wird.

4. Verfahren nach Anspruch 3 **dadurch gekennzeichnet, dass** die Fängermoleküle auf Beads fixiert sind.

5. Verfahren nach Anspruch 3 **dadurch gekennzeichnet, dass** die Fängermoleküle auf Nanomagneten immobilisiert sind.

6. Verfahren nach Anspruch 3 **dadurch gekennzeichnet, dass** die Fängermoleküle in einem Dialysesystem angeordnet sind.

7. Verfahren nach Anspruch 3 **dadurch gekennzeichnet, dass** der Träger für die Fänger-moleküle aus einem biokompatiblen Material ist.

8. Verfahren nach Anspruch 3 **dadurch gekennzeichnet, dass** die Träger Membrane, Filter, Filterschwämme, Beads, Stäbe, Seile, Säule, Hohlfasern sind.

9. Verfahren nach einem der vorangehenden Ansprüche **dadurch gekennzeichnet, dass** ein Kit zur selektiven Quantifizierung von A-Beta-Aggregaten und/oder zur Behandlung (in vitro) von Blut, Blutprodukten und/oder Organen enthaltend ein oder meh-rere der folgenden Komponenten:
- Substrat aus Glas das mit einem hydrophoben Stoff beschichtet ist;
- Standard;
Fängermolekül;
- Sonde;
- Substrat mit Fängermolekül;
- Lösungen;
- Puffer;
verwendet wird.

10. Verfahren nach einem der vorangehenden Ansprüche **dadurch gekennzeichnet, dass** die Verbindung aus Anspruch 1 als Fängermoleküle ex vivo über und/oder durch ein Organ geleitet werden.

11. Verfahren nach einem der vorangehenden Ansprüche **dadurch gekennzeichnet, dass** Amyloid-Beta-Oligomere enttoxifiziert werden.

12. Verwendung des D3D3 Peptids (SEQ ID NO:13) gemäß Anspruch 1 ex vivo als Fängermolekül für Amyloid-Beta-Oligomere.

13. Verwendung des D3D3 Peptids (SEQ ID NO:13) gemäß Anspruch 1 bei der ex vivo Behandlung von Blut, Blutprodukten und/oder Organen zur Befreiung und/oder Inaktivierung von Aß-Oligome-ren zur Prävention der Übertragung von Morbus Alzheimer.

14. Verwendung des D3D3 Peptids (SEQ ID NO:13) gemäß Anspruch 1 bei der ex vivo Behandlung von Morbus Alzheimer, Parkinson, Creutzfeldt Jakob Disease (CJD), Scrapie, bovine spongiforme Enzephalopatie (BSE), oder Diabetes.

## Claims

1. A method for the treatment outside of the human or animal body (in vitro, ex vivo) of blood, blood products and/or organs while amyloid-beta oligomers are removed and/or detoxified **characterized in that** the enantiomeric compound rprtrlhthmrrprtrlhthrnr (D3D3, SEQ ID NO:13) is used which binds to the amyloid-beta oligomers.

2. The method as claimed in claim 1, **characterized in that** before the treatmen,t the blood, the blood products or the organs are examined for amyloid-beta oligomers.

3. The method as claimed in any one of the preceding claims, **characterized in that** the compounds from claim 1 are arranged as capture molecules on a support, via which a sample containing the blood, the blood products and/or organ is passed.

4. The method as claimed in claim 3, **characterized in that** the capture molecules are fixed onto beads.

5. The method as claimed in claim 3, **characterized in that** the capture molecules are immobilized on nanomagnets.

6. The method as claimed in claim 3, **characterized in that** the capture molecules are arranged in a dialysis system.

7. The method as claimed in claim 3, **characterized in that** the support for the capture molecules is made of a biocompatible material.

8. The method as claimed in claim 3, **characterized in that** the supports are membranes, filters, filter sponges, beads, rods, cords, column, hollow fibers.

9. The method as claimed in any one of the preceding claims, **characterized in that** a kit is used for the selective quantification of A-beta aggregates and/or for treating (in vitro) blood, blood products and/or organs comprising one or more of the following components:
- substrate made of glass which is coated with a hydrophobic material;
- standard;
- capture molecule;
- probe;
- substrate with capture molecule;
- solutions;
- buffer.

10. The method as claimed in any one of the preceding claims, **characterized in that** the compound from claim 1 are passed as capture molecules ex vivo over and/or through an organ.

11. The method as claimed in any one of the preceding claims, **characterized in that** amyloid-beta oligomers are detoxified.

12. The use ex vivo of the D3D3 peptide (SEQ ID NO:13) as claimed in claim 1 as capture molecule for amyloid-beta oligomers.

13. The use of the D3D3 peptide, (SEQ ID NO:13) as claimed in claim 1 for the ex vivo treatment of blood, blood products and/or organs for the freeing and/or deactivation of Aß-oligomers for preventing the transmission of Alzheimer's disease.

14. The use of the D3D3 peptide (SEQ ID NO:13) as claimed in claim 1 for the ex vivo treating of Alzheimer's disease, Parkinson's disease, Creutzfeldt Jakob disease (CJD), scrapie, bovine spongiform encephalopathy (BSE), or diabetes.

## Revendications

1. Un procédé pour le traitement à l'extérieur du corps humain ou des animaux (in vitro, ex vivo) de sang, des produits sanguins et/ou des organes par l'extraction et/ou détoxification des oligomères bêta- amyloïdes, **caractérisé en ce que** le composition énantiomère rprtrlhthrnrrprtrlhthrnr (D3D3, SEQ ID NO:13) est appliqué que lie aux oligomères bêta- amyloïdes.

2. Procédé selon la revendication 1, **caractérisée en ce qu'**avant le traitement le sang, les produits sanguins ou les organes sont analysés aux oligomères bêta- amyloïdes.

3. Procédé selon l'une des revendications précédentes, **caractérisée en ce que** les compositions de la revendication 1 sont arrangées comme molécules receveurs sur un support, par lequel un échantillon , contenant le sang, les produits sanguins ou/ou les organes est conduit.

4. Procédé selon la revendication 3, **caractérisée en ce que** les molécules receveur sont fixées sur des perles.

5. Procédé selon la revendication 3, **caractérisée en ce que** les molécules receveur sont immobilisées sur des aimants nano.

6. Procédé selon la revendication 3, **caractérisée en ce que** les molécules receveur sont arrangées dans un système de dialyse.

7. Procédé selon la revendication 3, **caractérisée en ce que** le support pour des molécules receveur est fait du matériau biocompatible.

8. Procédé selon la revendication 3, **caractérisée en ce que** des supports sont des membranes, filtres, éponges filtrantes, perles, bâtons, cordes, colonnes, fibres creuses.

9. Procédé selon l'une des revendications précédentes, **caractérisée en ce qu'**un kit pour la quantification sélective des agrégats A-beta et/ou pour le traitement (in vitro) de sang, des produits sanguins et/ou des organes comprenant un ou plusieurs des components suivants:
- substrat fait de vitre lequel est revêtu d'un matériau hydrophobique;
- standard;
- molécule receveur;
- sonde;
- substrat avec une molécule receveur;
- solutions;
- tampons
est usé.

10. Procédé selon l'une des revendications précédentes, **caractérisée en ce que** la composition de la revendication 1 est conduite ex vivo sur et/ou par une organe comme molécule receveur.

11. Procédé selon l'une des revendications précédentes, **caractérisée en ce que** les oligomères bêta-amyloïde sont désintoxifiqués.

12. Utilisation du peptide D3D3 (SEQ ID NO:13) selon la revendication 1 ex vivo comme molécule receveur pour des oligomères bêta-amyloïde.

13. Utilisation du peptide D3D3 (SEQ ID NO:13) selon la revendication 1 pour le traitement ex vivo de sang, des produits sanguins et/ou des organes pour la déliberation et/ou déactivation des Aß-oligomères pour la prévention de la transmission de la maladie d' Alzheimer.

14. Utilisation de la peptide D3D3 (SEQ ID NO:13) selon la revendication 1 pour le traitement ex vivo de la maladie d' Alzheimer, maladie de Parkinson, maladie de Creutzfeldt Jakob , tremblante du mouton, encéphalopathie spongiforme ou diabète.
